# EUROPEAN PATENT APPLICATION

(11) **EP 4 523 703 A1**
(43) Date of publication of application: **19.03.2025**
(21) Application number: 23803601.6
(22) Date of filing: 11.05.2023
(51) Int. Cl.: A61K 45/00, A61K 31/18, A61K 31/381, A61K 31/404, A61K 31/4418, A61K 31/4439, A61K 31/4453, A61K 31/451, A61K 31/4545, A61K 31/4995, A61K 31/5025, A61K 31/517, A61P 1/16, A61P 1/18, A61P 11/00, A61P 13/12, G01N 33/15, G01N 33/50

(54) **PHARMACEUTICAL COMPOSITION FOR TREATING OR PREVENTING CYSTIC DISEASE**

(30) Priority: 11.05.2022 JP 2022078491
(71) Applicant: CHUGAI SEIYAKU KABUSHIKI KAISHA, Tokyo 115-8543 (JP)
(72) Inventor: ICHIDA, Yasuhiro, Gotemba-shi, Shizuoka 412-8513 (JP); IIDA, Manami, Gotemba-shi, Shizuoka 412-8513 (JP); MURAI, Atsuko, Gotemba-shi, Shizuoka 412-8513 (JP); HORIBA, Naoshi, Gotemba-shi, Shizuoka 412-8513 (JP)
(74) Representative: Vossius & Partner Patentanwälte Rechtsanwälte mbB
(86) International application number: PCT/JP2023/017706
(87) International publication number: WO 2023/219127

(57) **Abstract**

The present invention provides a pharmaceutical composition comprising a compound that inhibits a phosphate transporter as an active ingredient, for use in treating or preventing a cystic disease.

## Description

### TECHNICAL FIELD

The present invention relates to a pharmaceutical composition for treating or preventing a cystic disease, comprising a compound that inhibits a phosphate transporter as an active ingredient. The present invention also relates to a method for predicting a sensitivity of a patient having a cystic disease to a selective inhibitor of NaPi-IIb, and a method for treating or preventing a cystic disease.

### BACKGROUND ART

Cystic diseases include cystic kidney disease, pancreatic cystic disease, cystic liver, cystic lung disease, pancreatic cyst, or the like.

Polycystic kidney disease (PKD), one of the cystic diseases, is a hereditary disease in which many cysts are formed in the kidney. The growth of the cysts causes an increase in kidney volume and a decrease in kidney function (NPL 1).

So far, tolvaptan has been the only drug used to treat PKD, but side effects such as hepatotoxicity, polyuria, and the like have been reported (NPL 3). Thus, developments of new PKD treatments that reduce these problems have been desired.

Phosphorus is an element that is present in every cell and accounts for 1% of body weight, and plays an essential role in life support such as cell energy metabolism. The concentration of phosphorus in the blood is regulated by bone formation and bone resorption in addition to absorption from the gastrointestinal tract and excretion from the kidney, and is regulated to a constant concentration. Phosphorus absorption in the gastrointestinal tract is mainly performed by NaPi-IIb (SLC34A2), a phosphate transporter (NPLs 4 and 5). Phosphorus in the blood is filtered in the glomeruli of the kidney and the required amount is reabsorbed mainly by NaPi-IIa (SLC34A1) and NaPi-IIc (SLC34A3) (NPLs 4 and 6) in the renal tubules. The kidney plays a very important role in the regulation of phosphorus in vivo, and in patients with end-stage renal failure and dialysis having impaired renal function, phosphorus accumulates in the body, causing an increase in blood phosphorus concentration, that is, hyperphosphatemia.

Inhibiting NaPi-IIb, which plays a large role in phosphorus absorption in the gastrointestinal tract, is believed to be able to lead to suppression of phosphorus absorption in the gastrointestinal tract similarly to phosphorus adsorbents, and reduce the concentration of phosphorus in the blood (NPLs 5 and 7). To date, there have been some reports on NaPi-IIb inhibitors (PTLs 1 to 6, NPLs 8 and 9).

### CITATION LIST

### PATENT LITERATURE

PTL 1: International Publication No. WO2012/006475
PTL 2: International Publication No. WO2011/136269
PTL 3: International Publication No. WO2008/051980
PTL 4: International Publication No. WO2013/062065
PTL 5: International Publication No. WO2016/039458
PTL 6: International Publication No. WO2014/142273

### NON PATENT LITERATURE

NPL 1: Vervloe, M., Nat Rev Nephrol. 2019 Feb;15(2):70-72
NPL 2: Wu, M. et al., Kidney Dis 2016;2:128-135
NPL 3: Blair, H.A, et al., Drugs. 2019 Feb;79(3):303-313
NPL 4: Miyamoto, K. et al., J Pharm Sci. 2011 Sep; 100(9): 3719-30
NPL 5: Sabbagh, Y. et al., J Am Soc Nephrol. 2009 Nov; 20(11): 2348-58
NPL 6: Murer, H. et al., Pflugers Arch.2004 Feb;447(5):763-7
NPL 7: Ohi, A. et al., Am J Physiol Renal Physiol. 2011 Nov; 301(5): F1105-13
NPL 8: Tsuboi, Y., et al., Kidney International (2020) 98, 343-354
NPL 9: Maruyama, S. et al., Kidney International Reports (2021) 6, 675-684
NPL 10: Dixon EE., J Am Soc Nephrol. 2022 Feb;33(2):279-289

### SUMMARY OF INVENTION

### TECHNICAL PROBLEM

It is an object of the present invention to provide a medicament for treating or preventing a cystic disease.

### SOLUTION TO PROBLEM

The present inventors have studied considering the above problems, and as a result, have found that the phosphate transporter is highly expressed in the kidney tissue of patients with cystic disease. The present inventors have also found that compounds that inhibit phosphate transporters have an excellent effect in treating or preventing cystic diseases, and have completed the present invention. The present description encompasses the following disclosures of the invention.

[A-1] A pharmaceutical composition comprising a compound that inhibits a phosphate transporter as an active ingredient, for use in treating or preventing a cystic disease.

[A-2] A pharmaceutical composition for suppressing formation or expansion of a cyst, comprising a compound that inhibits a phosphate transporter as an active ingredient.

[A-3] The pharmaceutical composition according to [A-1] or [A-2], wherein the phosphate transporter is at least one selected from the group consisting of NaPi-IIa, NaPi-IIb, and NaPi-IIc.

[A-4] The pharmaceutical composition according to any of [A-1] to [A-3], wherein the phosphate transporter is NaPi-IIb.

[A-5] The pharmaceutical composition according to any of [A-1], [A-3] and [A-4], wherein the cystic disease is a disease in which NaPi-IIb is highly expressed in an organ developing the cystic disease.

[A-6] The pharmaceutical composition according to any of [A-1] and [A-3] to [A-5], wherein the cystic disease is a disease involving NaPi-IIb.

[A-7] The pharmaceutical composition according to any of [A-2] to [A-4], wherein NaPi-IIb is highly expressed in a cell of the cyst.

[A-8] The pharmaceutical composition according to any of [A-2] to [A-4] and [A-7], wherein the cyst is a cyst involving NaPi-IIb.

[A-9] The pharmaceutical composition according to any of [A-1] to [A-8], wherein the compound is at least one selected from the group consisting of a low-molecular compound, a polypeptide, and a polynucleotide.

[A-10] The pharmaceutical composition according to any of [A-1] to [A-9], wherein the compound is a low-molecular compound having a molecular weight of 2000 g/mol or less.

[A-11] The pharmaceutical composition according to any of [A-1] to [A-9], wherein the compound is a low-molecular compound having a molecular weight of 1000 g/mol or less.

[A-12] The pharmaceutical composition according to any of [A-1] to [A-9], wherein the compound is a polypeptide comprising an antibody.

[A-13] The pharmaceutical composition according to any of [A-1] to [A-9], wherein the compound is an anti-phosphate transporter antibody.

[A-14] The pharmaceutical composition according to any of [A-1] to [A-13], wherein the compound is at least one selected from the group consisting of an anti-NaPi-IIa antibody, an anti-NaPi-IIb antibody, and an anti-NaPi-IIc antibody.

[A-15] The pharmaceutical composition according to any of [A-1] to [A-9], wherein the compound is an anti-NaPi-IIb antibody.

[A-16] The pharmaceutical composition according to any of [A-1] to [A-9], wherein the compound is a polynucleotide selected from the group consisting of a ribozyme, an antisense molecule, an inhibitor oligonucleotide, an aptamer, a microRNA, and a small interfering RNA (siRNA).

[A-17] The pharmaceutical composition according to any of [A-1] to [A-9], wherein the compound is a polynucleotide selected from the group consisting of an antisense molecule, a microRNA, and a small interfering RNA (siRNA).

[A-18] The pharmaceutical composition according to any of [A-1] to [A-17], wherein the compound is at least one selected from the following (i) to (iii):
(i) a low-molecular compound having a molecular weight of 2000 g/mol or less;
(ii) an anti-NaPi-IIb antibody; and
(iii) a polynucleotide selected from the group consisting of an antisense molecule, a microRNA, and a small interfering RNA (siRNA).

[A-19] The pharmaceutical composition according to any of [A-1] to [A-18], wherein the compound is administered parenterally.

[A-20] The pharmaceutical composition according to any of [A-1] to [A-11] and [A-18], wherein the compound is administered orally.

[A-21] The pharmaceutical composition according to any of [A-1] to [A-20], wherein the compound is a compound that selectively inhibits the NaPi-IIb of the phosphate transporter.

[A-22] The pharmaceutical composition according to any of [A-1] to [A-21], wherein the compound is a compound that selectively inhibits the NaPi-IIb of the phosphate transporter and has a 50% phosphate uptake inhibition concentration (IC₅₀) ratio in a cell expressing NaPi-IIb or Pit1 (IC₅₀[Pit1]/IC₅₀[NaPi-IIb]) of 10 or more.

[A-23] The pharmaceutical composition according to any of [A-1] to [A-22], wherein the compound is a compound that selectively inhibits the NaPi-IIb of the phosphate transporter and has a 50% phosphate uptake inhibition concentration (IC₅₀) ratio in a cell expressing NaPi-IIb or Pit2 (IC₅₀[Pit2]/IC₅₀[NaPi-IIb]) of 10 or more.

[A-24] The pharmaceutical composition according to any of [A-1] to [A-23], wherein the compound has a 50% phosphate uptake inhibition concentration (IC₅₀) in a cell expressing the phosphate transporter of 10 µg/mol or less.

[A-25] The pharmaceutical composition according to any of [A-1] to [A-24], wherein the compound has a 50% phosphate uptake inhibition concentration (IC₅₀) in a cell expressing NaPi-IIb of 5 µg/mol or less.

[A-26] The pharmaceutical composition according to any of [A-1] to [A-25], wherein the compound has a 50% phosphate uptake inhibition concentration (IC₅₀) in a cell expressing human NaPi-IIb of 1 µg/mol or less.

[A-27] The pharmaceutical composition according to any of [A-1] to [A-26], wherein the compound is selected from the group consisting of compounds represented by formulas 1 to 11: 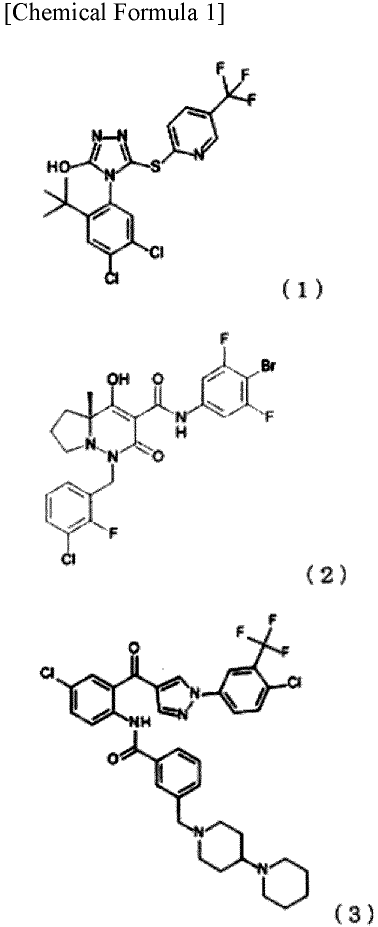 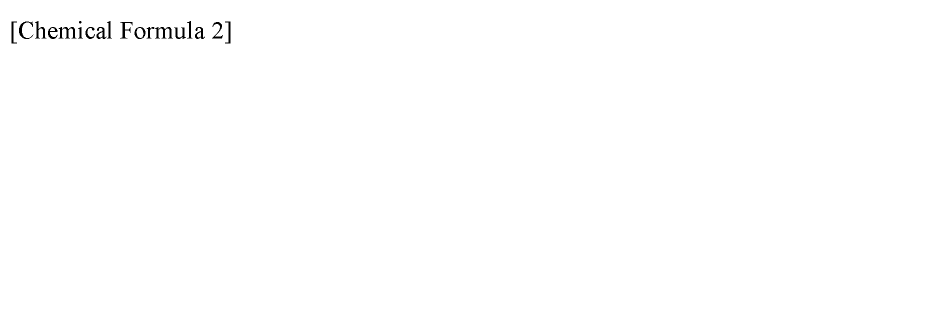 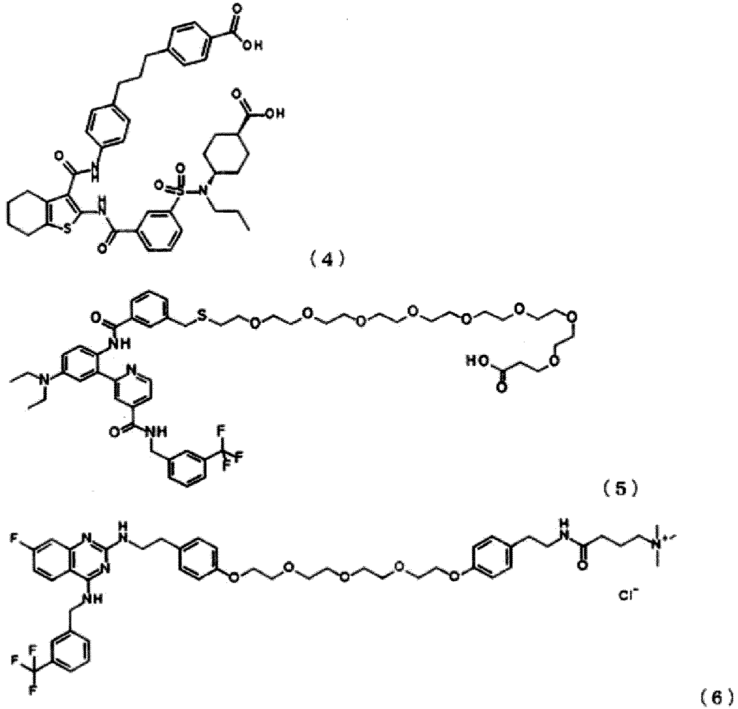 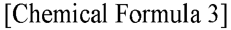 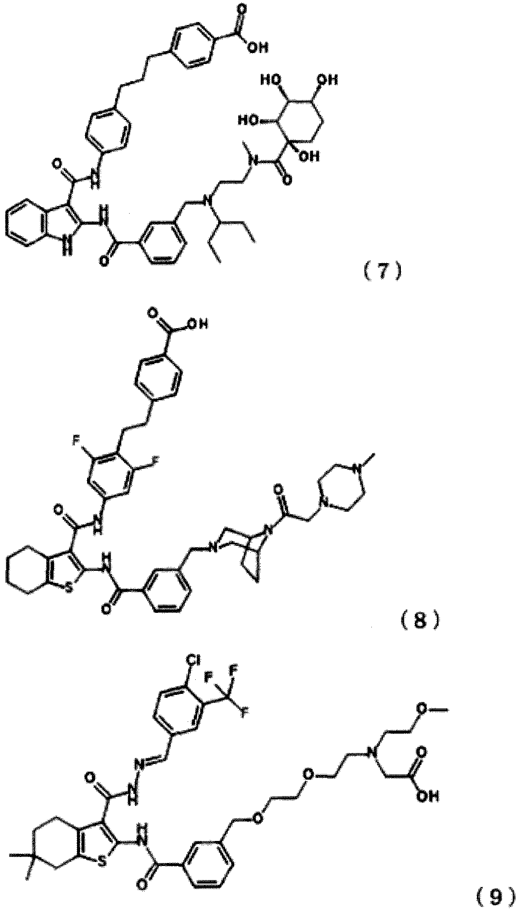 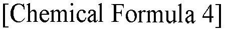 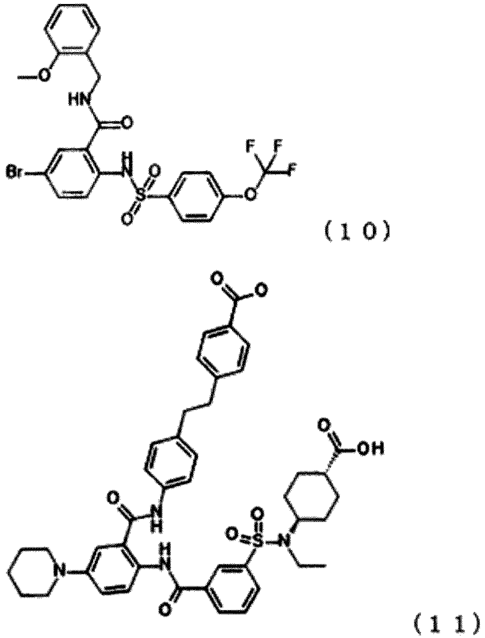 , or a salt thereof, or a solvate thereof.

[A-28] The pharmaceutical composition according to any of [A-1] to [A-26], wherein the compound is a compound selected from the group consisting of compounds represented by the formulas 1 to 4 above, or a salt thereof, or a solvate thereof.

[A-29] The pharmaceutical composition according to any of [A-1] to [A-28], wherein the cystic disease is a disease in a mammal.

[A-30] The pharmaceutical composition according to [A-29], wherein the mammal is a human.

[A-31] The pharmaceutical composition according to any of [A-1] to [A-30], wherein the cystic disease is selected from the group consisting of cystic kidney disease, polycystic liver disease, cystic lung disease, and pancreatic cyst.

[A-32] The pharmaceutical composition according to any of [A-2] to [A-30], wherein the cystic disease involves formation or expansion of a utricle in a kidney, a liver, a lung, or a pancreas.

[A-33] The pharmaceutical composition according to any of [A-1] to [A-32] for inhibiting a phosphate transporter expressed in a kidney, a liver, a lung, or a pancreas.

[A-34] The pharmaceutical composition according to any of [A-1] to [A-33] for use in treating or preventing cystic kidney disease.

[A-35] The pharmaceutical composition according to any of [A-1] to [A-34] for use in treating or preventing polycystic kidney disease.

[A-36] The pharmaceutical composition according to [A-35], wherein the polycystic kidney disease is autosomal dominant polycystic kidney disease.

[A-37] The pharmaceutical composition according to any of [A-1] to [A-36] for use in suppressing formation or expansion of a renal cyst.

[B-1] A method for treating or preventing a cystic disease, comprising administering an effective amount of a compound that inhibits a phosphate transporter to a subject in need thereof.

[B-2] A method for suppressing formation or expansion of a cyst, comprising administering an effective amount of a compound that inhibits a phosphate transporter to a subject in need thereof.

[B-3] The method according to [B-1] or [B-2], wherein the phosphate transporter is at least one selected from the group consisting of NaPi-IIa, NaPi-IIb, and NaPi-IIc.

[B-4] The method according to any of [B-1] to [B-3], wherein the phosphate transporter is NaPi-IIb.

[B-5] The method according to any of [B-1], [B-3] and [B-4], wherein the cystic disease is a disease in which NaPi-IIb is highly expressed in an organ developing the cystic disease.

[B-6] The method according to any of [B-1] and [B-3] to [B-5], wherein the cystic disease is a disease involving NaPi-IIb.

[B-7] The method according to any of [B-2] to [B-4], wherein NaPi-IIb is highly expressed in a cell of the cyst.

[B-8] The method according to any of [B-2] to [B-4] and [B-7], wherein the cyst is a cyst involving NaPi-IIb.

[B-9] The method according to any of [B-1] to [B-8], wherein the compound is at least one selected from the group consisting of a low-molecular compound, a polypeptide, and a polynucleotide.

[B-10] The method according to any of [B-1] to [B-9], wherein the compound is a low-molecular compound having a molecular weight of 2000 g/mol or less.

[B-11] The method according to any of [B-1] to [B-9], wherein the compound is a low-molecular compound having a molecular weight of 1000 g/mol or less.

[B-12] The method according to any of [B-1] to [B-9], wherein the compound is a polypeptide comprising an antibody.

[B-13] The method according to any of [B-1] to [B-9], wherein the compound is an anti-phosphate transporter antibody.

[B-14] The method according to any of [B-1] to [B-13], wherein the compound is at least one selected from the group consisting of an anti-NaPi-IIa antibody, an anti-NaPi-IIb antibody, and an anti-NaPi-IIc antibody.

[B-15] The method according to any of [B-1] to [B-9], wherein the compound is an anti-NaPi-IIb antibody.

[B-16] The method according to any of [B-1] to [B-9], wherein the compound is a polynucleotide selected from the group consisting of a ribozyme, an antisense molecule, an inhibitor oligonucleotide, an aptamer, a microRNA, and a small interfering RNA (siRNA).

[B-17] The method according to any of [B-1] to [B-9], wherein the compound is a polynucleotide selected from the group consisting of an antisense molecule, a microRNA, and a small interfering RNA (siRNA).

[B-18] The method according to any of [B-1] to [B-17], wherein the compound is at least one selected from the following (i) to (iii):
(i) a low-molecular compound having a molecular weight of 2000 g/mol or less;
(ii) an anti-NaPi-IIb antibody; and
(iii) a polynucleotide selected from the group consisting of an antisense molecule, a microRNA, and a small interfering RNA (siRNA).

[B-19] The method according to any of [B-1] to [B-18], wherein the compound is administered parenterally.

[B-20] The method according to any of [B-1] to [B-11] and [B-18], wherein the compound is administered orally.

[B-21] The method according to any of [B-1] to [B-20], wherein the compound is a compound that selectively inhibits the NaPi-IIb of the phosphate transporter.

[B-22] The method according to any of [B-1] to [B-21], wherein the compound is a compound that selectively inhibits the NaPi-IIb of the phosphate transporter and has a 50% phosphate uptake inhibition concentration (IC₅₀) ratio in a cell expressing NaPi-IIb or Pit1 (IC₅₀[Pit1]/IC₅₀[NaPi-IIb]) of 10 or more.

[B-23] The method according to any of [B-1] to [B-22], wherein the compound is a compound that selectively inhibits the NaPi-IIb of the phosphate transporter and has an IC₅₀ ratio (IC₅₀[Pit2]/IC₅₀[NaPi-IIb]) of 10 or more.

[B-24] The method according to any of [B-1] to [B-23], wherein the compound has a 50% phosphate uptake inhibition concentration (IC₅₀) in a cell expressing the phosphate transporter of 10 µg/mol or less.

[B-25] The method according to any of [B-1] to [B-24], wherein the compound has IC₅₀ of inhibitory activity against human NaPi-IIb of 5 µg/mol or less.

[B-26] The method according to any of [B-1] to [B-25], wherein the compound has IC₅₀ of inhibitory activity against human NaPi-IIb of 1 µg/mol or less.

[B-27] The method according to any of [B-1] to [B-20], wherein the compound is a compound selected from the group consisting of compounds represented by the formulas 1 to 11 above, or a salt thereof, or a solvate thereof.

[B-28] The method according to any of [B-1] to [B-20], wherein the compound is a compound selected from the group consisting of compounds represented by the formulas 1 to 4 above, or a salt thereof, or a solvate thereof.

[B-29] The method according to any of [B-1] to [B-28], wherein the subject is a mammal.

[B-30] The method according to [B-29], wherein the mammal is a human.

[B-31] The method according to any of [B-1] to [B-30], wherein the cystic disease is selected from the group consisting of cystic kidney disease, polycystic liver disease, cystic lung disease, and pancreatic cyst.

[B-32] The method according to any of [B-2] to [B-30], wherein the cystic disease involves formation or expansion of a utricle in a kidney, a liver, a lung, or a pancreas.

[B-33] The method according to any of [B-1] to [B-32], wherein the compound inhibits a phosphate transporter expressed in a kidney, a liver, a lung, or a pancreas.

[B-34] The method according to any of [B-1] to [B-33] for use in treating or preventing cystic kidney disease.

[B-35] The method according to any of [B-1] to [B-34] for treating or preventing polycystic kidney disease.

[B-36] The method according to [B-35], wherein the polycystic kidney disease is autosomal dominant polycystic kidney disease.

[B-37] The method according to any of [B-1] to [B-36] for suppressing formation or expansion of a renal cyst.

[C-1] Use of a compound that inhibits a phosphate transporter, in a manufacture of a medicament for treating or preventing a cystic disease.

[C-2] Use of a compound that inhibits a phosphate transporter, in a manufacture of a medicament for suppressing formation or expansion of a cyst.

[C-3] The use according to [C-1] or [C-2], wherein the phosphate transporter is at least one selected from the group consisting of NaPi-IIa, NaPi-IIb, and NaPi-IIc.

[C-4] The use according to any of [C-1] to [C-3], wherein the phosphate transporter is NaPi-IIb.

[C-5] The use according to any of [C-1], [C-3] and [C-4], wherein the cystic disease is a disease in which NaPi-IIb is highly expressed in an organ developing the cystic disease.

[C-6] The use according to any of [C-1] and [C-3] to [C-5], wherein the cystic disease is a disease involving NaPi-IIb.

[C-7] The use according to any of [C-2] to [C-4], wherein NaPi-IIb is highly expressed in a cell of the cyst.

[C-8] The use according to any of [C-2] to [C-4] and [C-7], wherein the cyst is a cyst involving NaPi-IIb.

[C-9] The use according to any of [C-1] to [C-8], wherein the compound is at least one selected from the group consisting of a low-molecular compound, a polypeptide, and a polynucleotide.

[C-10] The use according to any of [C-1] to [C-9], wherein the compound is a low-molecular compound having a molecular weight of 2000 g/mol or less.

[C-11] The use according to any of [C-1] to [C-9], wherein the compound is a low-molecular compound having a molecular weight of 1000 g/mol or less.

[C-12] The use according to any of [C-1] to [C-9], wherein the compound is a polypeptide comprising an antibody.

[C-13] The use according to any of [C-1] to [C-9], wherein the compound is an anti-phosphate transporter antibody.

[C-14] The use according to any of [C-1] to [C-13], wherein the compound is at least one selected from the group consisting of an anti-NaPi-IIa antibody, an anti-NaPi-IIb antibody, and an anti-NaPi-IIc antibody.

[C-15] The use according to any of [C-1] to [C-9], wherein the compound is an anti-NaPi-IIb antibody.

[C-16] The use according to any of [C-1] to [C-9], wherein the compound is a polynucleotide selected from the group consisting of a ribozyme, an antisense molecule, an inhibitor oligonucleotide, an aptamer, a microRNA, and a small interfering RNA (siRNA).

[C-17] The use according to any of [C-1] to [C-9], wherein the compound is a polynucleotide selected from the group consisting of an antisense molecule, a microRNA, and a small interfering RNA (siRNA).

[C-18] The use according to any of [C-1] to [C-17], wherein the compound is at least one selected from the following (i) to (iii):
(i) a low-molecular compound having a molecular weight of 2000 g/mol or less;
(ii) an anti-NaPi-IIb antibody; and
(iii) a polynucleotide selected from the group consisting of an antisense molecule, a microRNA, and a small interfering RNA (siRNA).

[C-19] The use according to any of [C-1] to [C-18], wherein the compound is administered parenterally.

[C-20] The use according to any of [C-1] to [C-11] and [C-18], wherein the compound is administered orally.

[C-21] The use according to any of [C-1] to [C-20], wherein the compound is a compound that selectively inhibits the NaPi-IIb of the phosphate transporter.

[C-22] The use according to any of [C-1] to [C-21], wherein the compound is a compound that selectively inhibits the NaPi-IIb of the phosphate transporter and has a 50% phosphate uptake inhibition concentration (IC₅₀) ratio in a cell expressing NaPi-IIb or Pit1 (IC₅₀[Pit1]/IC₅₀[NaPi-IIb]) of 10 or more.

[C-23] The use according to any of [C-1] to [C-22], wherein the compound is a compound that selectively inhibits the NaPi-IIb of the phosphate transporter and has an IC₅₀ ratio (IC₅₀[Pit2]/IC₅₀[NaPi-IIb]) of 10 or more.

[C-24] The use according to any of [C-1] to [C-23], wherein the compound has a 50% phosphate uptake inhibition concentration (IC₅₀) in a cell expressing the phosphate transporter of 10 µg/mol or less.

[C-25] The use according to any of [C-1] to [C-24], wherein the compound has IC₅₀ of inhibitory activity against human NaPi-IIb of 5 µg/mol or less.

[C-26] The use according to any of [C-1] to [C-25], wherein the compound has IC₅₀ of inhibitory activity against human NaPi-IIb of 1 µg/mol or less.

[C-27] The use according to any of [C-1] to [C-20], wherein the compound is a compound selected from the group consisting of compounds represented by the formulas 1 to 11 above, or a salt thereof, or a solvate thereof.

[C-28] The use according to any of [C-1] to [C-20], wherein the compound is a compound selected from the group consisting of compounds represented by the formulas 1 to 4 above, or a salt thereof, or a solvate thereof.

[C-29] The use according to any of [C-1] to [C-28], wherein the cystic disease is a disease in a mammal.

[C-30] The use according to [C-29], wherein the mammal is a human.

[C-31] The use according to any of [C-1] to [C-30], wherein the cystic disease is selected from the group consisting of cystic kidney disease, polycystic liver disease, cystic lung disease, and pancreatic cyst.

[C-32] The use according to any of [C-2] to [C-30], wherein the cystic disease involves formation or expansion of a utricle in a kidney, a liver, a lung, or a pancreas.

[C-33] The use according to any of [C-1] to [C-32] for inhibiting a phosphate transporter expressed in a kidney, a liver, a lung, or a pancreas.

[C-34] The use according to any of [C-1] to [C-33] for use in treating or preventing cystic kidney disease.

[C-35] The use according to any of [C-1] to [C-34] for use in treating or preventing polycystic kidney disease.

[C-36] The use according to [C-35], wherein the polycystic kidney disease is autosomal dominant polycystic kidney disease.

[C-37] The use according to any of [C-1] to [C-36] for use in suppressing formation or expansion of a renal cyst.

[D-1] A compound that inhibits a phosphate transporter, for use in treating or preventing a cystic disease.

[D-2] A compound that inhibits a phosphate transporter, for use in suppressing formation or expansion of a renal cyst.

[D-3] The compound according to [D-1] or [D-2], wherein the phosphate transporter is at least one selected from the group consisting of NaPi-IIa, NaPi-IIb, and NaPi-IIc.

[D-4] The compound according to any of [D-1] to [D-3], wherein the phosphate transporter is NaPi-IIb.

[D-5] The compound according to any of [D-1], [D-3] and [D-4], wherein the cystic disease is a disease in which NaPi-IIb is highly expressed in an organ developing the cystic disease.

[D-6] The compound according to any of [D-1] and [D-3] to [D-5], wherein the cystic disease is a disease involving NaPi-IIb.

[D-7] The compound according to any of [D-2] to [D-4], wherein NaPi-IIb is highly expressed in a cell of the cyst.

[D-8] The compound according to any of [D-2] to [D-4] and [D-7], wherein the cyst is a cyst involving NaPi-IIb.

[D-9] The compound according to any of [D-1] to [D-8], wherein the compound is at least one selected from the group consisting of a low-molecular compound, a polypeptide, and a polynucleotide.

[D-10] The compound according to any of [D-1] to [D-9], wherein the compound is a low-molecular compound having a molecular weight of 2000 g/mol or less.

[D-11] The compound according to any of [D-1] to [D-9], wherein the compound is a low-molecular compound having a molecular weight of 1000 g/mol or less.

[D-12] The compound according to any of [D-1] to [D-9], wherein the compound is a polypeptide comprising an antibody.

[D-13] The compound according to any of [D-1] to [D-9], wherein the compound is an anti-phosphate transporter antibody.

[D-14] The compound according to any of [D-1] to [D-13], wherein the compound is at least one selected from the group consisting of an anti-NaPi-IIa antibody, an anti-NaPi-IIb antibody, and an anti-NaPi-IIc antibody.

[D-15] The compound according to any of [D-1] to [D-9], wherein the compound is an anti-NaPi-IIb antibody.

[D-16] The compound according to any of [D-1] to [D-9], wherein the compound is a polynucleotide selected from the group consisting of a ribozyme, an antisense molecule, an inhibitor oligonucleotide, an aptamer, a microRNA, and a small interfering RNA (siRNA).

[D-17] The compound according to any of [D-1] to [D-9], wherein the compound is a polynucleotide selected from the group consisting of an antisense molecule, a microRNA, and a small interfering RNA (siRNA).

[D-18] The compound according to any of [D-1] to [D-17], wherein the compound is at least one selected from the following (i) to (iii):
(i) a low-molecular compound having a molecular weight of 2000 g/mol or less;
(ii) an anti-NaPi-IIb antibody; and
(iii) a polynucleotide selected from the group consisting of an antisense molecule, a microRNA, and a small interfering RNA (siRNA).

[D-19] The compound according to any of [D-1] to [D-18], wherein the compound is administered parenterally.

[D-20] The compound according to any of [D-1] to [D-11] and [D-18], wherein the compound is administered orally.

[D-21] The compound according to any of [D-1] to [D-20], wherein the compound is a compound that selectively inhibits the NaPi-IIb of the phosphate transporter.

[D-22] The compound according to any of [D-1] to [D-21], wherein the compound is a compound that selectively inhibits the NaPi-IIb of the phosphate transporter and has a 50% phosphate uptake inhibition concentration (IC₅₀) ratio in a cell expressing NaPi-IIb or Pit1 (IC₅₀[Pit1]/IC₅₀[NaPi-IIb]) of 10 or more.

[D-23] The compound according to any of [D-1] to [D-22], wherein the compound is a compound that selectively inhibits the NaPi-IIb of the phosphate transporter and has an IC₅₀ ratio (IC₅₀[Pit2]/IC₅₀[NaPi-IIb]) of 10 or more.

[D-24] The compound according to any of [D-1] to [D-23], wherein the compound has a 50% phosphate uptake inhibition concentration (IC₅₀) in a cell expressing the phosphate transporter of 10 µg/mol or less.

[D-25] The compound according to any of [D-1] to [D-24], wherein the compound has IC₅₀ of inhibitory activity against human NaPi-IIb of 5 µg/mol or less.

[D-26] The compound according to any of [D-1] to [D-25], wherein the compound has IC₅₀ of inhibitory activity against human NaPi-IIb of 1 µg/mol or less.

[D-27] The compound according to any of [D-1] to [D-20], wherein the compound is a compound selected from the group consisting of compounds represented by the formulas 1 to 11 above, or a salt thereof, or a solvate thereof.

[D-28] The compound according to any of [D-1] to [D-20], wherein the compound is a compound selected from the group consisting of compounds represented by the formulas 1 to 4 above, or a salt thereof, or a solvate thereof.

[D-29] The compound according to any of [D-1] to [D-28], wherein the cystic disease is a disease in a mammal.

[D-30] The compound according to [D-29], wherein the mammal is a human.

[D-31] The compound according to any of [D-1] to [D-30], wherein the cystic disease is selected from the group consisting of cystic kidney disease, polycystic liver disease, cystic lung disease, and pancreatic cyst.

[D-32] The compound according to any of [D-2] to [D-30], wherein the cystic disease involves formation or expansion of a utricle in a kidney, a liver, a lung, or a pancreas.

[D-33] The compound according to any of [D-1] to [D-32] for inhibiting a phosphate transporter expressed in a kidney, a liver, a lung, or a pancreas.

[D-34] The compound according to any of [D-1] to [D-33] for use in treating or preventing cystic kidney disease.

[D-35] The compound according to any of [D-1] to [D-34] for use in treating or preventing polycystic kidney disease.

[D-36] The compound according to [D-35], wherein the polycystic kidney disease is autosomal dominant polycystic kidney disease.

[D-37] The compound according to any of [D-1] to [D-36] for use in suppressing formation or expansion of a renal cyst.

[E-1] A method for predicting a benefit of a treatment with an agent in a patient having a cystic disease, the method comprising:
confirming whether NaPi-IIb is expressed in a cell collected from an organ of the patient in which a cyst has been formed; and
determining the patient as a patient who would benefit from the treatment when the expression is confirmed,
wherein the agent is an inhibitor of NaPi-IIb.

[E-2] The method according to [E-1], wherein the cystic disease is cystic kidney disease.

[E-3] The method according to [E-1] or [E-2], wherein the cystic disease is polycystic kidney disease.

[E-4] The method according to [E-3], wherein the polycystic kidney disease is autosomal dominant polycystic kidney disease.

[E-5] The method according to any of [E-1] to [E-4], wherein the inhibitor is a low-molecular compound, a polynucleotide, and a polypeptide.

[E-6] The pharmaceutical composition according to any of [E-1] to [E-5], wherein the inhibitor is a low-molecular compound having a molecular weight of 2000 g/mol or less.

[E-7] The pharmaceutical composition according to any of [E-1] to [E-6], wherein the inhibitor is a low-molecular compound having a molecular weight of 1000 g/mol or less.

[E-8] The method according to any of [E-1] to [E-5], wherein the inhibitor comprises an antibody or a binding fragment thereof.

[E-9] The method according to any of [E-1] to [E-5], wherein the inhibitor is an anti-phosphate transporter antibody.

[E-10] The method according to any of [E-1] to [E-5], wherein the inhibitor is at least one selected from the group consisting of an anti-NaPi-IIa antibody, an anti-NaPi-IIb antibody, and an anti-NaPi-IIc antibody.

[E-11] The method according to any of [E-1] to [E-5], wherein the inhibitor is an anti-NaPi-IIb antibody.

[E-12] The method according to any of [E-1] to [E-5], wherein the inhibitor is a polynucleotide selected from the group consisting of a ribozyme, an antisense molecule, an inhibitor oligonucleotide, an aptamer, a microRNA, and a small interfering RNA (siRNA).

[E-13] The pharmaceutical composition according to any of [E-1] to [E-5], wherein the inhibitor is a polynucleotide selected from the group consisting of an antisense molecule, a microRNA, and a small interfering RNA (siRNA).

[E-14] The pharmaceutical composition according to any of [E-1] to [E-5], wherein the inhibitor is at least one selected from the following (i) to (iii):
(i) a low-molecular compound having a molecular weight of 2000 g/mol or less;
(ii) an anti-NaPi-IIb antibody; and
(iii) a polynucleotide selected from the group consisting of an antisense molecule, a microRNA, and a small interfering RNA (siRNA).

[E-15] The method according to any of [E-1] to [E-14], wherein the inhibitor is administered parenterally.

[E-16] The method according to any of [E-1] to [E-7] and [E-14], wherein the inhibitor is administered orally.

[E-17] The method according to any of [E-1] to [E-16], wherein the inhibitor is a compound that selectively inhibits the NaPi-IIb of the phosphate transporter.

[E-18] The method according to any of [E-1] to [E-17], wherein the compound is a compound that selectively inhibits the NaPi-IIb of the phosphate transporter and has a 50% phosphate uptake inhibition concentration (IC₅₀) ratio in a cell expressing NaPi-IIb or Pit1 (IC₅₀[Pit1]/IC₅₀[NaPi-IIb]) of 10 or more.

[E-19] The method according to any of [E-1] to [E-18], wherein the compound is a compound that selectively inhibits the NaPi-IIb of the phosphate transporter and has a 50% phosphate uptake inhibition concentration (IC₅₀) ratio in a cell expressing NaPi-IIb or Pit2 (IC₅₀[Pit2]/IC₅₀[NaPi-IIb]) of 10 or more.

[E-20] The method according to any of [E-1] to [E-19], wherein the compound has a 50% phosphate uptake inhibition concentration (IC₅₀) in a cell expressing the phosphate transporter of 10 µg/mol or less.

[E-21] The method according to any of [E-1] to [E-20], wherein the compound has a 50% phosphate uptake inhibition concentration (IC₅₀) in a cell expressing NaPi-IIb of 5 µg/mol or less.

[E-22] The method according to any of [E-1] to [E-21], wherein the compound has a 50% phosphate uptake inhibition concentration (IC₅₀) in a cell expressing human NaPi-IIb of 1 µg/mol or less.

[E-23] The method according to any of [E-1] to [E-19], wherein the inhibitor is a compound selected from the group consisting of compounds of the formulas 1 to 11 above.

[E-24] The method according to any of [E-1] to [E-19], wherein the inhibitor is a compound selected from the group consisting of compounds of the formulas 1 to 4 above.

[E-25] The method according to any of [E-1] to [E-24], wherein the cystic disease is a disease in a mammal.

[E-26] The method according to [E-25], wherein the mammal is a human.

[E-27] The method according to any of [E-1] to [E-26], wherein the cystic disease is cystic kidney disease, polycystic liver disease, cystic lung disease, or pancreatic cyst.

[E-28] The method according to any of [E-2] to [E-26], wherein the cyst is a cyst developed in a kidney, a liver, a lung, or a pancreas.

[E-29] The method according to any of [E-1] to [E-28], wherein the cell is a renal cell contained in a biological sample from the patient.

[F-1] A pharmaceutical composition comprising a compound that inhibits a phosphate transporter as an active ingredient, for use in suppressing renal cystogenesis in a subject at risk for autosomal dominant polycystic kidney disease.

[F-2] The pharmaceutical composition according to [F-1], wherein the subject has a confirmed familial occurrence of autosomal dominant polycystic kidney disease.

[F-3] The pharmaceutical composition according to [F-1] or [F-2], wherein either of the parents of the subject is a patient having chromosomal dominant polycystic kidney disease.

[F-4] The pharmaceutical composition according to any of [F-1] to [F-3], wherein the subject has a defect in PKD1 or PKD2.

[F-5] The pharmaceutical composition according to any of [F-1] to [F-4], wherein the subject has a defect in PKD1.

[F-6] The pharmaceutical composition according to any of [F-1] to [F-5], wherein the subject is 30 years of age or older.

[F-7] The pharmaceutical composition according to any of [F-1] to [F-6], wherein the subject is confirmed to have three or more cysts in each kidney on both sides by CT, MRI, or ultrasound tomography.

[F-8] The pharmaceutical composition according to any of [F-1] to [F-7], wherein the subject has been diagnosed as having autosomal dominant polycystic kidney disease.

[F-9] The pharmaceutical composition according to any of [F-1] to [F-8], wherein the phosphate transporter is at least one selected from the group consisting of NaPi-IIa, NaPi-IIb, and NaPi-IIc.

[F-10] The pharmaceutical composition according to any of [F-1] to [F-9], wherein the phosphate transporter is NaPi-IIb.

[F-11] The pharmaceutical composition according to any of [F-1] to [F-10], wherein NaPi-IIb is expressed in a kidney cell of a subject.

[F-12] The pharmaceutical composition according to any of [F-1] to [F-11], wherein the compound is a compound that inhibits a phosphate transporter expressed in a kidney.

[F-13] The pharmaceutical composition according to any of [F-1] to [F-12], wherein the compound is a low-molecular compound, a polynucleotide, and a polypeptide.

[F-14] The pharmaceutical composition according to any of [F-1] to [F-13], wherein the compound is a low-molecular compound having a molecular weight of 2000 g/mol or less.

[F-15] The pharmaceutical composition according to any of [F-1] to [F-13], wherein the compound is a low-molecular compound having a molecular weight of 1000 g/mol or less.

[F-16] The pharmaceutical composition according to any of [F-1] to [F-13], wherein the compound comprises an antibody or a binding fragment thereof.

[F-17] The pharmaceutical composition according to any of [F-1] to [F-13], wherein the compound is an anti-phosphate transporter antibody.

[F-18] The pharmaceutical composition according to any of [F-1] to [F-13], wherein the compound is at least one selected from the group consisting of an anti-NaPi-IIa antibody, an anti-NaPi-IIb antibody, and an anti-NaPi-IIc antibody.

[F-19] The pharmaceutical composition according to any of [F-1] to [F-13], wherein the compound is an anti-NaPi-IIb antibody.

[F-20] The pharmaceutical composition according to any of [F-1] to [F-13], wherein the compound is a polynucleotide selected from the group consisting of a ribozyme, an antisense molecule, an inhibitor oligonucleotide, an aptamer, a microRNA, and a small interfering RNA (siRNA).

[F-21] The pharmaceutical composition according to any of [F-1] to [F-20], wherein the compound is administered parenterally.

[F-22] The pharmaceutical composition according to any of [F-1] to [F-15], wherein the compound is administered orally.

[F-23] The pharmaceutical composition according to any of [F-1] to [F-18], wherein the compound is a compound that selectively inhibits the NaPi-IIb of the phosphate transporter.

[F-24] The pharmaceutical composition according to any of [F-1] to [F-23], wherein the compound is a compound that selectively inhibits the NaPi-IIb of the phosphate transporter and has a 50% phosphate uptake inhibition concentration (IC₅₀) ratio in a cell expressing NaPi-IIb or Pit2 (IC₅₀[Pit2]/IC₅₀[NaPi-IIb]) of 10 or more.

[F-25] The pharmaceutical composition according to any of [F-1] to [F-24], wherein the compound has a 50% phosphate uptake inhibition concentration (IC₅₀) in a cell expressing the phosphate transporter of 10 µg/mol or less.

[F-26] The pharmaceutical composition according to any of [F-1] to [F-25], wherein the compound has a 50% phosphate uptake inhibition concentration (IC₅₀) in a cell expressing NaPi-IIb of 5 µg/mol or less.

[E-27] The pharmaceutical composition according to any of [F-1] to [F-26], wherein the compound has a 50% phosphate uptake inhibition concentration (IC₅₀) in a cell expressing human NaPi-IIb of 1 µg/mol or less.

[F-28] The pharmaceutical composition according to any of [F-1] to [F-27], wherein the compound is a compound selected from the group consisting of compounds represented by the formulas 1 to 11 above, or a salt thereof, or a solvate thereof.

[F-29] The pharmaceutical composition according to any of [F-1] to [F-28], wherein the compound is a compound selected from the group consisting of compounds represented by the formulas 1 to 4 above, or a salt thereof, or a solvate thereof.

[G-1] A method of screening for a compound for use in treating or preventing a disease selected from cystic kidney disease, polycystic liver disease, cystic lung disease, and pancreatic cyst, the method comprising measuring inhibitory activity against a phosphate transporter.

[G-2] A method of screening for a compound that suppresses cyst formation or cyst expansion in an organ of a mammal, the method comprising measuring an inhibitory activity against a phosphate transporter.

[G-3] The method according to [G-2], wherein the organ is a pancreas.

[G-4] The method according to any of [G-1] to [G-3], wherein the phosphate transporter is at least one selected from the group consisting of NaPi-IIa, NaPi-IIb, and NaPi-IIc.

[G-5] The method according to any of [G-1] to [G-4], wherein the phosphate transporter is NaPi-IIb.

[G-6] The method according to any of [G-1] to [G-5], wherein the disease is cystic kidney disease.

[G-7] The method according to [G-6], wherein the cystic kidney disease is polycystic kidney disease.

[G-8] The method according to [G-7], wherein the polycystic kidney disease is autosomal dominant polycystic kidney disease.

[G-9] The method according to any of [G-1] to [G-8], wherein the disease is a disease in a mammal.

[G-10] The method according to [G-9], wherein the mammal is a human.

[G-11] The method according to any of [G-1] to [G-10], wherein the inhibitory activity is a selective inhibitory activity against NaPi-IIb relative to Pit1 or Pit2.

### ADVANTAGEOUS EFFECTS OF INVENTION

In one aspect of the present invention, a pharmaceutical composition useful for treating or preventing a cystic disease and a medicament for treating or preventing a cystic disease are provided. In another aspect of the present invention, a method for predicting a benefit of a treatment with an agent in a patient having a cystic disease is provided.

### BRIEF DESCRIPTION OF DRAWINGS

Fig. 1a is a diagram showing a HE staining image and NaPi-IIb expression levels in a kidney tissue of an ADPKD patient.
Fig. 1b is a diagram showing a HE staining image and NaPi-IIb expression levels in a kidney tissue of an ADPKD patient.
Fig. 1c is a diagram showing a HE staining image and NaPi-IIb expression levels in a kidney tissue of an ADPKD patient.
Fig. 1d is a diagram showing a HE staining image and NaPi-IIb expression levels in a kidney tissue of an ADPKD patient.
Fig. 1e is a diagram showing a HE staining image and NaPi-IIb expression levels in a kidney tissue of an ADPKD patient.
Fig. If is a diagram showing a HE staining image and NaPi-IIb expression levels in a kidney tissue of an ADPKD patient.
Fig. 2a is a diagram showing a HE staining image and NaPi-IIb expression levels in a kidney tissue of a B6 mouse. (a) is the results of B6 mouse, (b) is the results of a PC1 Conditional KO mouse, and (c) is the results of a Pcy mouse.
Fig. 2b is a diagram showing a HE staining image and NaPi-IIb expression levels in a kidney tissue of a PC1 conditional knockout mouse.
Fig. 2c is a diagram showing a HE staining image and NaPi-IIb expression levels in a kidney tissue of a Pcy mouse.
Fig. 3a shows the results of evaluating the effects of tolvaptan and compound 4 on renal volume in a pcy mouse.
Fig. 3b shows the results of evaluating the effects of tolvaptan and compound 4 on blood urea nitrogen (UN) concentrations in a pcy mouse.
Fig. 3c shows the results of evaluating the effects of tolvaptan and compound 4 on body weight in a pcy mouse.
Fig. 3d shows the results of evaluating the effects of tolvaptan and compound 4 on urine volume in pcy mouse.
Fig. 4 is three-dimensional photographed images of (a) stimulating agent and compound 4-non added well, (b) stimulating agent-added and compound 4-non added well, and (c) stimulating agent and compound 4-added well in cultured wells of a kidney tissue of a human ADPKD patient.
Fig. 5a shows the results of evaluating the effects of tolvaptan and compound 4 on renal volume in a PC1 conditional knockout mouse.
Fig. 5b shows the results of evaluating the effects of tolvaptan and compound 4 on body weight in a pcy mouse.
Fig. 5c shows the results of evaluating the effects of tolvaptan and compound 4 on urine volume in a pcy mouse.

### DESCRIPTION OF EMBODIMENTS

Embodiments of the present invention will be described. However, the present invention is not limited to the following embodiments. The medicament for use in a treatment or prevention and the method for treating or preventing according to the present invention may be administered or applied to a human. As used herein, the "to" that indicates a numerical range includes values of both ends thereof. For example, "A to B" means the numerical range of A or more and B or less. As used herein, the term "about", when used in combination with a numeric value, means the value range of +10% and -10% of the numeric value. As used herein, the term "and/or" is meant to include every combination of the terms "and" and "or" appropriately combined. Specifically, for example, the term "A, B, and/or C" includes the following seven variations: (i) A, (ii) B, (iii) C, (iv) A and B, (v) A and C, (vi) B and C, and (vii) A, B, and C.

The present invention provides a pharmaceutical composition comprising a compound that inhibits a phosphate transporter as an active ingredient, for use in treating or preventing a cystic disease or for suppressing formation or expansion of a cyst.

In one aspect of the present invention, the "phosphate transporter" is not particularly limited as long as it is involved in phosphorus metabolism in vivo. Examples thereof include transporters involved in reabsorption from the kidney, absorption from the gastrointestinal tract, and migration to and from bone and other tissues, and transporters involved in maintaining phosphorus metabolic homeostasis in vivo. The phosphate transporter include a sodium-dependent phosphate transporter. The sodium-dependent phosphate transporter is sometimes referred to as a sodium-phosphate cotransporter and encompasses phosphate transporters of type I, II, or III.

In one aspect of the present invention, examples of the phosphate transporter include NaPi-IIa, NaPi-IIb, NaPi-IIc, Pit1, and Pit2, and preferred examples include NaPi-IIb.

In one aspect of the present invention, the compound that inhibits a phosphate transporter is not particularly limited as long as it has an inhibitory activity against the phosphate transporter or inhibits the expression of the phosphate transporter. As used herein, the compound that inhibits a phosphate transporter may also be referred to as a phosphate transporter inhibitor.

In one aspect of the present invention, the "treatment" means that the expansion of cyst in the organ of an individual is suppressed, the number of cyst cells is decreased, the proliferation of cyst cells is suppressed, the volume of cyst is decreased, the weight of cyst is decreased, the metastasis of cyst cells is suppressed, or various symptoms caused by cyst are ameliorated by the pharmaceutical composition of the present invention. Also, in some embodiments, the "prevention" in the present invention means a formation of cyst is suppressed, an increase in the number of cyst cells due to the re-proliferation of decreased cyst cells is prevented, a re-proliferation of cyst cells whose proliferation is suppressed is prevented, a re-increase of decreased cyst size is prevented, or a macroscopic reappearance of cyst that has macroscopically disappeared (or healed) due to topical treatment is prevented.

As used herein, the "pharmaceutical composition" means a mixture comprising particular ingredients in predetermined amounts or proportions.

The pharmaceutical composition may comprise, in addition to an active ingredient, a pharmaceutically acceptable carrier. As used herein, the term "pharmaceutically acceptable carrier" means one or more solid or liquid excipient diluents or encapsulating materials suitable for administration to a mammal. As used herein, the term "pharmaceutically acceptable" means that it is usable as a medicament in terms of efficacy, safety, or the like.

Examples of additives that can be used as the pharmaceutically acceptable carrier include sugars such as lactose, glucose, and sucrose; starches such as corn starch and potato starch; cellulose and derivatives such as sodium carboxymethyl cellulose, ethyl cellulose, and methyl cellulose; tragacanth gum powder; malt; gelatin; talc; solid lubricants such as stearic acid and magnesium stearate; calcium sulfate; vegetable oils such as peanut oil, cottonseed oil, sesame oil, olive oil, corn oil, and cocoa oil; polyvalent alcohols such as propylene glycol, glycerin, sorbitol, mannitol, and polyethylene glycol; alginate; emulsifiers such as TWEEN(R); wetting agents such as lecithin; coloring agents; flavoring agents; tableting agents; stabilizing agents; antioxidants; preservatives; pyrogen-free water; aqueous isotonic salt solutions; and phosphate buffers.

In one aspect of the present invention, a method of identifying a target molecule of an agent for treating or preventing a particular disease is provided. The target molecule can be identified, for example, by identifying a gene suitable as a target of an agent by a method comprising the following steps.
(1) identifying a gene that is a factor of a disease: the gene that is a factor of a disease can be identified by analyzing a differentially expressed gene (DEG) in tissues of a normal group and a pathologic group.
(2) identifying a gene suitable as a target of an agent from the gene identified in (1): the gene suitable as a target of an agent can be identified from the gene that is a factor of the disease by combining multiple gene selection criteria. Examples of the gene selection criteria for selecting a gene suitable as a target of an agent include criteria that they are not genes related to innate immunity, acquired immunity, immune cells, and inflammation, criteria that they are not fibrosis-related genes associated with chronic inflammation, and criteria that they are genes related to membrane proteins. It is preferred that all of these gene selection criteria are met.

In one aspect of the present invention, the expression of NaPi-IIb can be confirmed, for example, according to the method described in NPL 10. More specifically, the expression can be confirmed by the methods described below.
1) A tissue containing a cyst is cut out from an organ and frozen blocks are made by OCT embedding.
2) The frozen blocks are sliced and put on slides for Visium Gene Expression, and HE staining was performed.
3) After obtaining the HE staining image, cDNA is generated and next-generation sequence analysis is performed.
4) Sequence analysis of short read sequence library is performed for cDNA.
5) The result of sequence analysis is reflected in the HE staining image.
6) The number of each gene in each spot calculated by the sequence analysis is corrected by the number of housekeeping genes and the number of nucleic acids in each spot to obtain a value of the expression level of each gene.

In one aspect of the present invention, high expression of NaPi-IIb refers to, for example, the case where NaPi-IIb is expressed in 10% or more, 20% or more, 30% or more, 40% or more, 50% or more, 60% or more, 70% or more, 80% or more, or 90% or more of the area of the entire organ tissue image in a HE staining image of the organ tissue reflecting the result of the sequence analysis obtained by the above method.

In one aspect of the present invention, a disease involving NaPi-IIb means a cystic disease the development, progression and/or continuation of which involves NaPi-IIb. The "cystic disease the formation, progression and/or continuation of which involves NaPi-IIb" includes not only a cystic disease the formation, progression and/or continuation of which directly involves NaPi-IIb, but also of which indirectly involves NaPi-IIb. The cystic disease involving NaPi-IIb is not particularly limited to, but may mean, for example, a cystic disease the formation, progression and/or continuation of which involves an increase in NaPi-IIb.

In one aspect of the present invention, a cyst involving NaPi-IIb means a cyst the formation, expansion, progression and/or continuation of which involves NaPi-IIb. The "cyst the formation, expansion, progression and/or continuation of which involves NaPi-IIb" includes not only a cyst the formation, expansion, progression and/or continuation of which directly involves NaPi-IIb, but also of which indirectly involves NaPi-IIb. The cyst involving NaPi-IIb is not particularly limited to, but may mean, for example, a cyst the formation, expansion, progression and/or continuation of which involves an increase in NaPi-IIb.

In one aspect of the present invention, the compound that inhibits a phosphate transporter is not particularly limited and may be a known compound and may be a compound identified by screening as described below, but preferably at least one selected from the group consisting of a low-molecular compound, a polypeptide, and a polynucleotide.

The polypeptide includes not only a full-length polypeptide encoded by a gene, but also a fragment thereof, a chemically synthesized polypeptide, a cyclic polypeptide, and a glycopeptide. The polypeptide also includes an antibody and an antigenic peptide, and the antibody may be a polyclonal antibody or a monoclonal antibody. The antibody includes, in addition to a complete antibody, an antibody fragment such as Fab, Fab', F(ab')₂, Fv, scFv, sc(Fv)₂, dsFv, a diabody, and the like, a multimer thereof, a low-molecular antibody in which a variable region of an antibody is bound, and an antibody-drug conjugate (ADC). The antibody is preferably an anti-phosphate transporter antibody, more preferably at least one selected from the group consisting of an anti-NaPi-IIa antibody, an anti-NaPi-IIb antibody, and an anti-NaPi-IIc antibody, and further preferably an anti-NaPi-IIb antibody.

Examples of the polynucleotide include a ribozyme, an antisense molecule, an inhibitor oligonucleotide, an aptamer, a microRNA, and a small interfering RNA (siRNA), and preferred examples include an antisense molecule, a microRNA, and siRNA. The polynucleotide includes not only a full-length polynucleotide, but also a fragment thereof and a synthesized polynucleotide. The unit of molecular weight in the present disclosure is "g/mol" (hereinafter, the unit of molecular weight may be omitted in the present specification). According to one aspect of the present invention, the compound is a compound having a molecular weight of 2000 g/mol or less, 1800 g/mol or less, 1600 g/mol or less, 1400 g/mol or less, 1200 g/mol or less, 1000 g/mol or less, 800 g/mol or less, 600 g/mol or less, 400 g/mol or less, or 200 g/mol or less. Specific examples thereof include the compounds described in WO2012/006475, WO2011/136269, WO2013/062065, WO2013/082756, WO2013/082751 and WO2013/129435, salts thereof, or solvates thereof. More specific examples include the following compounds represented by formulas 1 to 11, or pharmaceutically acceptable salts thereof.

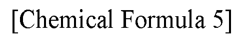

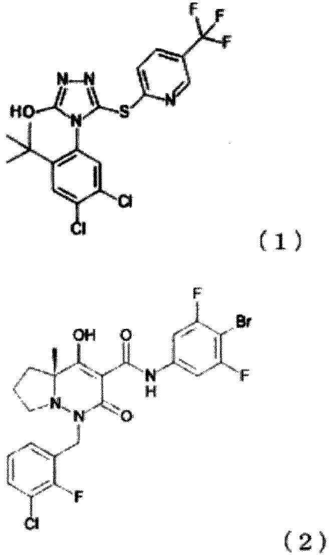

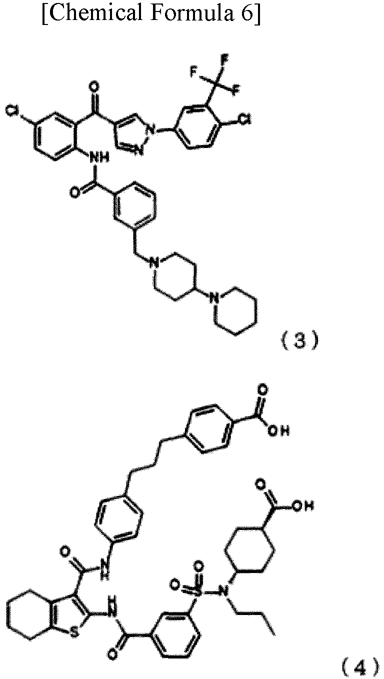

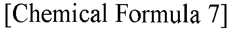

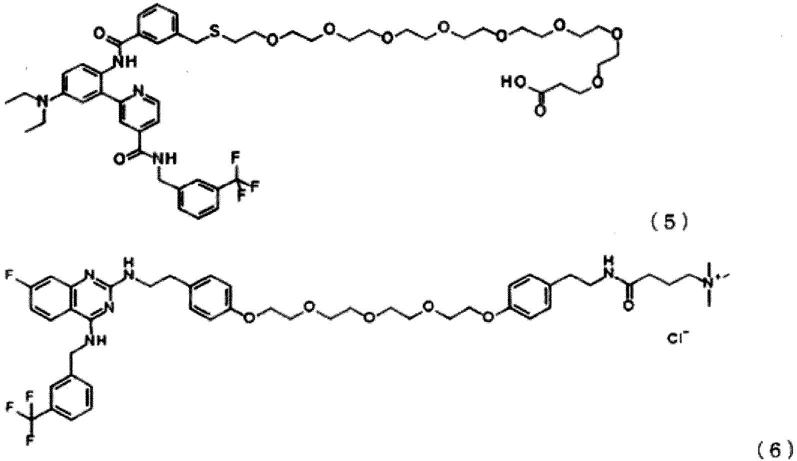

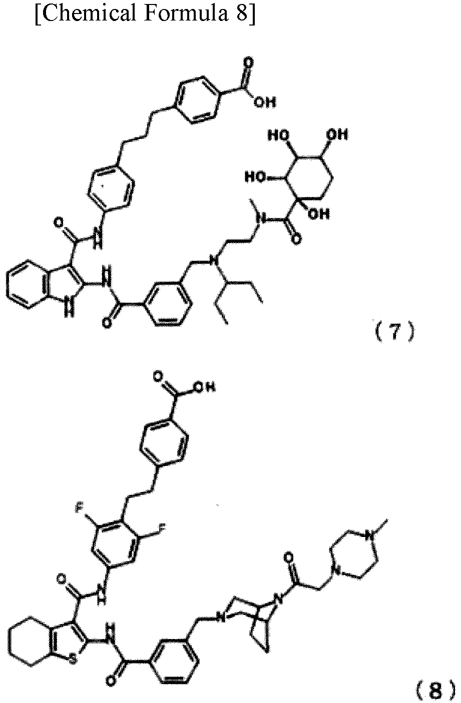

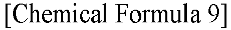

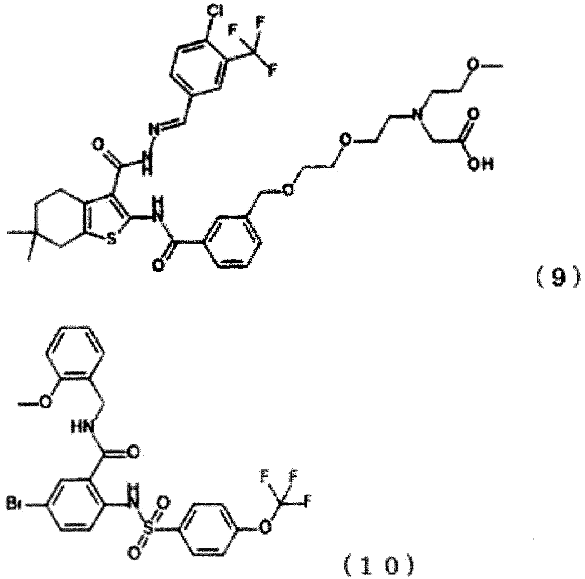

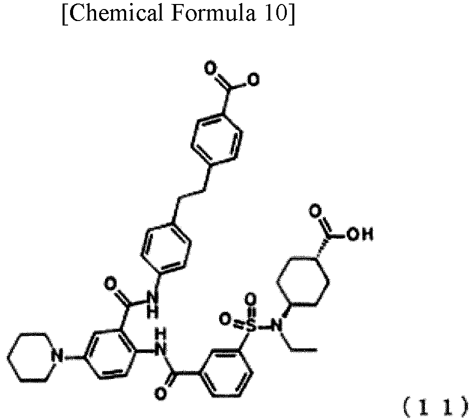

When using the pharmaceutical composition of the present invention, the administration method thereof may be an orally method or a parenterally method. Examples of the parenterally administration method include rectal, intravenous, intramuscular, subcutaneous, intracisternal, intravaginal, intraperitoneal, intravesical, or topical (infusion, powder, ointment, gel, or cream) administrations and inhalation (intraoral or nasal spray). Examples of dosage form thereof include a tablet, a capsule, a granule, a powder, a pill, aqueous and non-aqueous oral solutions and suspensions, and a parenteral solution filled in a container adapted to be subdivided into individual dosages. The dosage forms can also be adapted to a variety of administration methods encompassing modified release formulations such as subcutaneous grafting.

The above formulations are manufactured by well-known methods using additives such as an excipient, a lubricant (coating agent), a binder, a disintegrant, a stabilizer, a flavoring agent, and a diluent.

Examples of the excipient include starches such as starch, potato starch, and corn starch, lactose, crystalline cellulose, and calcium hydrogen phosphate.

Examples of the coating agent include ethyl cellulose, hydroxypropyl cellulose, hydroxypropyl methyl cellulose, shellac, talc, carnauba wax, and paraffin.

Examples of the binder include polyvinylpyrrolidone, macrogol, and compounds similar to the excipients.

Examples of the disintegrant include compounds similar to the excipient and chemically modified starches and celluloses such as croscarmellose sodium, carboxymethyl starch sodium, and crosslinked polyvinylpyrrolidone.

Examples of the stabilizer include parahydroxybenzoates such as methyl paraben and propyl paraben; alcohols such as chlorobutanol, benzyl alcohol, and phenylethyl alcohol; benzalkonium chloride; phenols such as phenol and cresol; thimerosal; dehydroacetic acid; and sorbic acid.

Examples of the flavoring agent include a sweetener, an acidulant, and a perfume, which are commonly used.

Examples of solvents for producing a liquid agent include ethanol, phenol, chlorocresol, purified water, and distilled water.

Examples of surfactant or emulsifier include polyoxyl 40 stearate and lauromacrogol.

In one aspect of the present invention, when a pharmaceutical composition is used, the amount used varies depending on the symptoms, age, body weight, relative health conditions, presence of other medications, administration method, or the like. For example, for patients (warm-blooded animals, especially humans), the generally effective amount is preferably 1 to 20 mg per kg of body weight per day, more preferably 1 to 10 mg per kg of body weight, as an active ingredient (a compound described herein used as an active ingredient) in the case of an oral agent, and the daily amount used is preferably in the range of 60 to 1200 mg for an adult patient having a normal weight. However, the amount is not necessarily limited to these values.

In one aspect of the present invention, the inhibitory activity of a compound that inhibits a phosphate transporter can be confirmed by known measurement methods. Here, the indicator to confirm the inhibitory activity may be a 50% phosphate uptake inhibition concentration (IC₅₀) in a cell expressing a phosphate transporter, for example, as measured by the method described in NPL 8. That is, it can be measured by the following method.
1) Human NaPi-IIb-expressing plasmids, human PiT1-expressing plasmids, or human PiT2-expressing plasmids are transfected into CHO cells.
2) The medium is replaced with buffer A (145 mM choline chloride, 3 mM KCl, 1 mM CaCl₂, 0.5 mM MgCl₂, 5 mM glucose, 5 mM MES, (pH 6.5)), and then replaced with buffer B (145 mM NaCl, 3 mM KCl, 1 mM CaCl₂, 0.5 mM MgCl₂, 5 mM glucose, 5 mM MES (pH 6.5)) to which a test compound is added at the final concentration of 0.01, 0.03, 0.1, 0.3, 1, 3, 10, or 30 µM, buffer A to which DMSO is added, or buffer B to which DMSO is added.
3) After a certain time, a 1/20 amount of buffer A containing ³³PO₄ is added and reacted at room temperature.
4) After washing with ice-cooled buffer A, a liquid scintillator is added, and the ³³PO₄ uptake amount is measured. The inhibition rate can be determined by the following formula. Inhibition rate (%) = (1 - (33PO4 uptake amount of test compound-added buffer B-treated well - 33PO4 uptake amount of DMSO-added buffer A-treated well)/(33PO4 uptake amount of DMSO-added buffer B-treated well - 33PO4 of DMSO-added buffer A-treated well)) x 100
5) The 50% ³³PO₄-uptake inhibition concentrations (IC₅₀) (µM) in a cell expressing human NaPi-IIb, human PiT1, and human PiT2 are calculated from a straight line through two points sandwiching a 50% inhibition rate.

In one aspect of the present invention, the 50% phosphate uptake inhibition concentration (IC₅₀) in a cell expressing a phosphate transporter is 10 µM or less, 5 µM or less, or 1 µM or less. In one aspect of the present invention, the 50% phosphate uptake inhibition concentration (IC₅₀) in a cell expressing NaPi-IIb is 10 µM or less, 5 µM or less, or 1 µM or less.

In one aspect of the present invention, a compound that inhibits a phosphate transporter is a compound that selectively inhibits NaPi-IIb, and, for example, may be a compound that selectively inhibits NaPi-IIb relative to Pit1 or Pit2. Selectively inhibiting NaPi-IIb means, for example, a case in which a 50% phosphate uptake inhibition concentration (IC₅₀) ratio in a cell expressing Pit1, Pit2, or NaPi-IIb (IC₅₀[Pit1]/IC₅₀[NaPi-IIb]) and/or (IC₅₀[Pit2]/IC₅₀[NaPi-IIb]) is 10 or more, 100 or more, or 500 or more.

In one aspect of the present invention, the compound that inhibits the phosphate transporter is a compound having IC₅₀[Pit1]/IC₅₀[NaPi-IIb] and/or IC₅₀[Pit2]/IC₅₀[NaPi-IIb] of 10 or more, 100 or more, or 500 or more.

In one aspect of the present invention, the compound used as an active ingredient in the pharmaceutical composition can be a salt thereof or a solvate thereof. The salt of the compound described herein can be a pharmaceutically acceptable salt. The salt of the compound described herein include an acid addition salt and a base addition salt. Examples of the acid addition salt include hydrochloride, hydrobromide, hydroiodide, phosphate, phosphonate, sulfate, or the like; sulfonates such as methane sulfonate, ethanesulfonate, benzenesulfonate, and p-toluenesulfonate; and carboxylates such as acetate, citrate, malate, tartrate, succinate, salicylate, maleate, fumarate, benzoate, malonate, glycolate, oxalate, glucuronate, adipate, glutarate, ketoglutarate, and hippurate. Examples of the base addition salt include alkali metal salts such as a sodium salt and a potassium salt; alkaline earth metal salts such as a magnesium salt and a calcium salt; ammonium salts such as an ammonium salt, an alkylammonium salt, a dialkylammonium salt, a trialkylammonium salt, and a tetraalkylammonium salt; and amino acid salts such as a lysine salt, an arginine salt, a glycine salt, a valine salt, a threonine salt, a serine salt, a proline salt, and an alanine salt. These salts are produced by bringing the compound in contact with an acid or base that can be used in the manufacture of a pharmaceutical product.

In one aspect of the present invention, the solvate refers to one in which a compound, together with a solvent, forms a single molecular assembly, and is not particularly limited as long as the solvate is formed with a solvent acceptable for ingestion along with administration of a medicament. Specific examples of the solvate include not only a hydrate, an alcohol hydrate (such as ethanol hydrate, methanol hydrate, 1-propanol hydrate, 2-propanol hydrate, or the like), a solvate formed with a single solvent such as dimethyl sulfoxide, but also a solvate formed with a plurality of solvents per one molecule of compound, or a solvate formed with a plurality of types of solvents per one molecule of compound. When the solvent is water, the solvate is called a hydrate.

The compounds described herein and salts thereof used as active ingredients in pharmaceutical compositions can be present as a number of tautomers, for example, keto and enol forms, imine and enamine forms, and a mixture thereof. The tautomer may be present in a solution as a mixture of tautomers. In a solid form, one tautomer is usually predominant. Although one tautomer may be described, the present invention includes all tautomers of the compounds of the present invention. The compound, a salt thereof, or a solvate thereof described herein include all their stereoisomers (e.g., enantiomers, diastereomers (cis and trans geometric isomers)), racemates of the isomers, and other mixtures. The compound may have, for example, one or more asymmetric points, and the present invention includes racemic mixtures, diastereomeric mixtures, and enantiomers of such compounds.

The compounds described herein used as an active ingredient in a pharmaceutical composition may comprise a non-naturally occurring percentage of isotopic atom in one or more atoms constituting such a compound. Compounds substituted with an isotope in a ratio different from the ratio of isotope in nature by substituting any atom in the compound with an atom having the same atomic number (proton number) and a different mass number (the sum of the number of protons and neutrons), i.e., compounds labeled with an isotopic atom, are also included in the present invention. Examples of the isotope element contained in the compound herein include a hydrogen atom, a carbon atom, a nitrogen atom, an oxygen atom, a phosphorus atom, a sulfur atom, a fluorine atom, and a chlorine atom, and they include ²H, ³H, ¹³C, ¹⁴C, ¹⁵N, ¹⁷O, ¹⁸O, ³¹P, ³²P, ³⁵S, ¹⁸F , ³⁶Cl, and the like, respectively. The compounds labeled with an isotopic atom are useful as therapeutic or prophylactic agents, research reagents (e.g., assay reagents), and diagnostic agents (e.g., in vivo imaging diagnostics). For the compounds as used herein, all the compounds containing radioactive or non-radioactive isotopic elements in any proportions are encompassed within the scope of the present invention. The compounds labeled with an isotopic atom can be produced by using reagents or solvents containing the corresponding isotopic atoms in a manner similar to the methods for producing unlabeled compounds.

The compound described herein used as an active ingredient in a pharmaceutical composition may be in the form of a prodrug. As used herein, the "prodrug" means a derivative of a compound described herein that is used as an active ingredient in a pharmaceutical composition after administration under physiological conditions by enzymatically or non-enzymatically degradation to convert into the compound or a pharmaceutically acceptable salt thereof. The prodrug may be inactive when administered to a patient, but is converted in vivo and present as the compound having an inhibitory activity against a phosphate transporter.

For example, a prodrug is converted into a desired drug form at a specific pH or by the action of an enzyme. Typical prodrugs are compounds that produce free acids in vivo and compounds that have a hydrolyzable ester group. Examples of such a hydrolyzable ester group include, but are not limited to, groups represented by the formula -COORx, wherein Rx is selected from C₁₋₄ alkyl, C₂₋₇ alkanoyloxymethyl, 1-(C₄₋₉ alkanoyloxy)ethyl, 1-methyl-1-(C₅₋₁₀ alkanoyloxy)-ethyl, (C₃₋₆ alkoxy)carbonyloxymethyl, 1-[(C₄₋₇ alkoxy)carbonyloxy]ethyl, 1-methyl-1-[(C₅₋₈ alkoxy)carbonyloxy]ethyl, N-[(C₃₋₉ alkoxy)carbonyl]aminomethyl, 1-(N-[(C₄₋₁₀ alkoxy)carbonyl]amino)ethyl, 3-phthalidyl, 4-crotonolactonyl, gamma-butyrolacton-4-yl, [N,N-di(C₁₋₂ alkyl)amino]C₂₋₃ alkyl (e.g., N,N-dimethylaminoethyl), (carbamoyl)C₁₋₂ alkyl, [N,N-di(C₁₋₂ alkyl)carbamoyl]C₁₋₂ alkyl, (piperidino)C₂₋₃ alkyl, (pyrrolidino)C₂₋₃ alkyl, or (morpholino)C₂₋₃ alkyl.

The subject to which the compound, the salt or the solvate of the present disclosure is administered is an animal, preferably a mammal (e.g., a mouse, a rat, a rabbit, a dog, a monkey (e.g., a cynomolgus monkey) or a human), particularly preferably a human. The human may be an adult (of 18 years of age or older) or a child (under 18 years of age). The child is preferably, e.g., of 6 months of age or older.

In one aspect of the present invention, a pharmaceutical composition comprising a compound that inhibits a phosphate transporter can be used in a prevention or treatment of a cystic disease or in suppressing formation or expansion of a cyst. Examples of the cystic disease include cystic kidney disease, polycystic liver disease, cystic lung disease, and pancreatic cyst. The formation or expansion of a cyst may occur, for example, in a kidney, a liver, a lung, or a pancreas.

In one aspect of the present invention, a pharmaceutical composition comprising a compound that inhibits a phosphate transporter can be used in a treatment or prevention of cystic kidney disease including polycystic kidney disease, autosomal dominant polycystic kidney disease (ADPKD), or the like, or in suppressing formation or expansion of a renal cyst. In addition to the diseases described above, the cystic kidney diseases include, for example, multiple simple renal cyst, renal tubular acidosis, multicystic kidney, multicystic dysplastic kidney, multilocular cysts of the kidney, medullary cystic disease of the kidney, juvenile nephronophthisis, acquired cystic disease of the kidney, and autosomal recessive polycystic kidney disease.

Polycystic kidney disease can be diagnosed, for example, by confirming the presence of multiple cysts in the kidneys on both sides by imaging (such as CT, MRI, or ultrasound tomography). In one aspect of the present invention, polycystic kidney disease is diagnosed when three or more, or five or more, cysts are confirmed in each kidney on both sides.

In one aspect of the present invention, the "effective amount" means a dose of a phosphate transporter inhibitor effective for treating or preventing a disease, particularly a cystic disease in the present invention, in an individual. Examples of the effective amount include, but are not limited to, a dose in the range of 0.0001 mg to 1000 mg, preferably 0.001 mg to 100 mg, and further preferably 0.01 to 50 mg per kg of body weight per dose, for example, every 1 to 10 weeks, preferably every 1 to 8 weeks, and even more preferably every 1 to 4 weeks.

In one aspect of the present invention, the following aspects are provided.
(1) A compound that inhibits NaPi-IIb for use in treating or preventing cystic kidney disease in a mammal, the compound being selected from the group consisting of the following (i) to (iii):
   (i) a low-molecular compound having a molecular weight of 2000 g/mol or less;
   (ii) an anti-NaPi-IIb antibody; and
   (iii) a polynucleotide selected from the group consisting of an antisense molecule, a microRNA, and a small interfering RNA (siRNA).
(2) A compound that inhibits NaPi-IIb for use in suppressing renal cystogenesis in a mammal, the compound being selected from the group consisting of the following (i) to (iii):
   (i) a low-molecular compound having a molecular weight of 2000 g/mol or less;
   (ii) an anti-NaPi-IIb antibody; and
   (iii) a polynucleotide selected from the group consisting of an antisense molecule, a microRNA, and a small interfering RNA (siRNA).

In one aspect of the present invention, a method for predicting a benefit of a treatment with an agent in a patient having a cystic disease, comprising confirming whether NaPi-IIb is expressed in a cell collected from an organ of the patient having a cystic disease in which a cyst has been formed is provided. Herein, the agent is an inhibitor of NaPi-IIb.

In one aspect of the present invention, a method for predicting a benefit of a treatment with an agent in a patient having cystic kidney disease, comprising confirming whether NaPi-IIb is expressed in a kidney cell collected from the patient having cystic kidney disease is provided. Herein, the agent is an inhibitor of NaPi-IIb. As the kidney cell collected from the patient having cystic kidney disease, a cell obtained, for example, by renal biopsy can be used, and specifically, a cell obtained by needle biopsy under ultrasound guidance or open kidney biopsy can be used.

In another aspect of the present invention, a pharmaceutical composition comprising a compound that inhibits a phosphate transporter as an active ingredient, for use in suppressing renal cystogenesis is provided. Renal cysts can be diagnosed, for example, by confirming them by image examining (such as ultrasound, CT, or MRI). In one aspect of the present invention, the pharmaceutical composition is used in a subject having a risk of autosomal dominant polycystic kidney disease until the onset of cystic kidney disease. In one aspect of the present invention, the pharmaceutical composition is used to suppress further renal cystogenesis, i.e., to suppress progression of the disease, even after the onset of cystic kidney disease.

In one aspect of the present invention, a compound that inhibits a phosphate transporter or a composition comprising the compound is administered to a subject having a confirmed familial occurrence of autosomal dominant polycystic kidney disease. Here, the familial occurrence is confirmed by the presence or absence of patients among parents, siblings, grandfathers, grandmothers, uncles, aunts, cousins, and the like. In one subject of the present invention, a compound that inhibits a phosphate transporter is administered to a subject whose either parent is a patient of chromosomal dominant polycystic kidney disease.

In one aspect of the present invention, a compound that inhibits a phosphate transporter or a composition comprising the compound is administered to a subject having a defect in PKD1 or PKD2. Here, the defect in PKD1 or PKD2 can be confirmed in a cell obtained from the subject by methods known to those skilled in the art. In one aspect of the present invention, the subject has a defect in PKD1.

The age of the subject to which a compound that inhibits a phosphate transporter or a composition comprising the compound is administered is not particularly limited, and may be, for example, 15 years of age or younger, and 16 years of age or older, 20 years of age or older, 25 years of age or older, 30 years of age or older, 35 years of age or older, 40 years of age or older, 45 years of age or older, 50 years of age or older, 60 years of age or older, or 70 years of age or older. In one aspect of the present invention, the compound or composition is administered to a subject of 30 years of age or older.

The cystic disease described herein or the formation or expansion of cysts can be diagnosed or confirmed by examination methods known to those skilled in the art, such as CT, MRI, or ultrasound tomography. In one aspect of the present invention, a compound that inhibits a phosphate transporter or a composition comprising the compound is administered to a subject confirmed to have one or two or more cysts in a particular organ, e.g., a kidney, a liver, a lung, or a pancreas. In one aspect of the present invention, the subject of administration is a subject confirmed to have one or two or more cysts in a kidney. Specifically, the compound or composition is administered to a subject confirmed to have three or more cysts in each kidney on both sides by CT, MRI, or ultrasound tomography.

In one aspect of the present invention, a method of screening for a compound for use in treating or preventing a disease selected from cystic disease, polycystic liver disease, cystic lung disease, or/and pancreatic cyst, the method comprising measuring an inhibitory activity against a phosphate transporter is provided. In one aspect of the present invention, a method of screening for a compound that suppresses cyst formation or cyst expansion in an organ of a mammal, the method comprising measuring an inhibitory activity against a phosphate transporter is also provided. These screening methods are hereinafter sometimes referred to as "method of screening for a compound".

A compound that inhibits the phosphate transporter, screened for in the method of screening for a compound described above can be used to provide a pharmaceutical composition for treating or preventing a disease involving the phosphate transporter, selected from cystic diseases, polycystic liver disease, cystic lung disease, or/and pancreatic cyst and cystic kidney disease, the pharmaceutical composition containing a compound that inhibits the phosphate transporter as an active ingredient.

The test compounds applied to the method of screening for a compound used as an active ingredient in a pharmaceutical composition are not particularly limited, and examples thereof include at least one selected from the group consisting of a low-molecular compound, a polypeptide, and a polynucleotide listed above as the compound that inhibits a phosphate transporter described above. More specific examples of the test compounds include synthetic low-molecular compound libraries, gene library expression products, peptide libraries, siRNAs, antibodies, bacterial release materials, extracts and culture supernatants of cells (microorganisms, plant cells, animal cells), purified or partially-purified polypeptides, marine organisms, extracts from plants or animals, and random phage peptide display libraries. The test compounds may also be derivatives of a known phosphate transporter.

The method of screening for a compound described above may comprise selecting a compound having an inhibitory activity against a phosphate transporter. The inhibitory activity against a phosphate transporter in the method of screening for a compound described above can be measured by known methods. The indicator to confirm the inhibitory activity may be a 50% phosphate uptake inhibition concentration (IC₅₀) in a cell expressing a phosphate transporter as described above.

In one aspect of the present invention, the following screening methods are provided.
(1) A method of screening for a compound for use in treating or preventing cystic kidney disease in a mammal, the method comprising measuring inhibitory activity against NaPi-IIb, and selecting a compound having an inhibitory activity against NaPi-IIb.
(2) A method of screening for a compound that suppresses cyst formation or cyst expansion in an organ of a mammal, the method comprising measuring inhibitory activity against NaPi-IIb, and selecting a compound having an inhibitory activity against NaPi-IIb, in an aspect of the present invention.
(3) A method of screening for a compound that suppresses cyst formation or cyst expansion in a kidney of a mammal, the method comprising measuring inhibitory activity against NaPi-IIb, and selecting a compound having an inhibitory activity against NaPi-IIb, in an aspect of the present invention.

Hereinafter, the present invention will be described in more detail using Examples, but the present invention is not limited to these Examples. All starting materials and reagents were obtained from commercial suppliers, or synthesized by known methods.

### EXAMPLES

### Pharmacological Test Examples

### Test Example 1: Evaluation of ³³PO₄ uptake inhibition in a cell expressing human NaPi-IIb, human PiT1, or human PiT2

For transient expression, human NaPi-IIb expression plasmids, human PiT1 expression plasmids, human PiT2 expression plasmids, and Fugene HD Transfection (Promega Corporation) were added to CHO cells using a 96-well plate, and the plate was incubated in a CO₂ incubator for 16 hours. The medium was replaced with buffer A (145 mM choline chloride, 3 mM KCl, 1 mM CaCl₂, 0.5 mM MgCl₂, 5 mM glucose, 5 mM MES, (pH 6.5)), and then replaced with buffer B (145 mM NaCl, 3 mM KCl, 1 mM CaCl₂, 0.5 mM MgCl₂, 5 mM glucose, 5 mM MES (pH 6.5)) to which a test compound was added at the final concentration of 0.01, 0.03, 0.1, 0.3, 1, 3, 10, or 30 µM, buffer A to which DMSO was added, or buffer B to which DMSO was added. After a certain time, a 1/20 amount of buffer A containing ³³PO₄ (PerkinElmer Japan G.K.) was added and reacted at room temperature. After washing with ice-cooled buffer A, a liquid scintillator (MicroScint^{™}-20, item number 6013621, PerkinElmer Japan G.K.) was added, and the ³³PO₄ uptake amount was measured with the top count (Top Count NXT, PerkinElmer Japan G.K.). The inhibition rate was determined by the following formula. Here, a certain time was set to 10 minutes. The phrase "a 1/20 amount of buffer A containing ³³PO₄ (PerkinElmer Japan G.K.) was added" refers to "buffer A containing ³³PO₄ (PerkinElmer Japan G.K.) was added in an amount of 1/ 20 of the amount of solution filling the medium".

Inhibition rate (%) = (1 - (33PO4 uptake amount of test compound-added buffer B-treated well - 33PO4 uptake amount of DMSO-added buffer A-treated well)/(33PO4 uptake amount of DMSO-added buffer B-treated well - 33PO4 of DMSO-added buffer A-treated well)) x 100

The 50% ³³PO₄-uptake inhibition concentrations (IC₅₀) (µM) in a cell expressing human NaPi-IIb, human PiT1, and human PiT2 were calculated from a straight line through two points sandwiching 50% inhibition rate. The results are shown in Table 1. It should be noted that compounds 1 and 2 were not measured for human PiT1 and human PiT2, which was indicated as "-" in Table 1.

**[Table 1]**

| [Table 1: Results of Test Example 1] | | | | | |
|---|---|---|---|---|---|
| Compound No. | Structural formula | Compound name | Uptake Inhibition IC₅₀ (µM) | | |
| | | | NaPi-IIb | Pit1 | Pit2 |
| 1 | 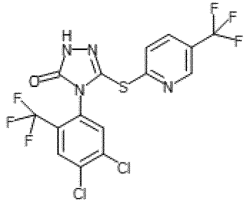 | 4-[4,5-Dichloro-2-(trifluoromethyl)phenyl]-3-[5-(trifluoromethyl)pyridin-2-yl]sulfanyl-1H-1,2,4-triazol-5-one | 7.9 | - | - |
| 2 | 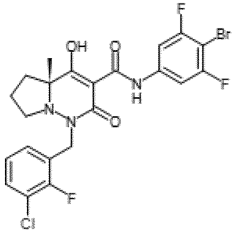 | (4aR)-N-(4-Bromo-3,5-difluorophenyl)-1-[(3-chloro-2-fluorophenyl)methyl]-4-hydroxy-4a-methyl-2-oxo-6,7-dihydro-5H-pyrrolo[1,2-b]pyridazine-3-carboxamide | 2.9 | - | - |
| 3 | 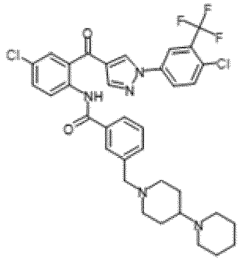 | N-[4-Chloro-2-[1-[4-chloro-3-(trifluoromethyl)phenyl]pyra zole-4-carbonyl]phenyl]-3-[(4-piperidin-1-ylpiperidin-1-yl)methyl]benzamide | 3.8 | >30 | >30 |
| 4 | 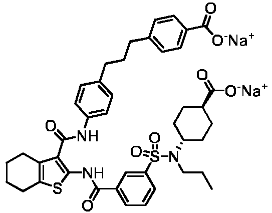 | Disodium; 4-[3-[4-[[2-[[3-[(4-carboxylatocyclohexyl)-propylsulfamoyl]benzoyl]am ino]-4,5,6,7-tetrahydro-1-benzothiophene-3-carbonyl]amino]phenyl]prop yl]benzoic acid | 0.031 | >30 | >30 |

The results of the above tests confirmed the inhibitory effect of each test compound on ³³PO₄ uptake of human NaPi-IIb-expressing cells.

### Test Example 2: Evaluation of NaPi-IIb expression levels in kidney tissue of human ADPKD patient

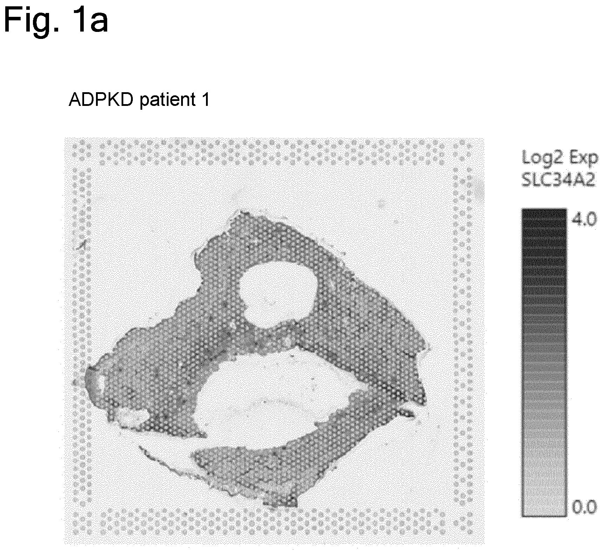

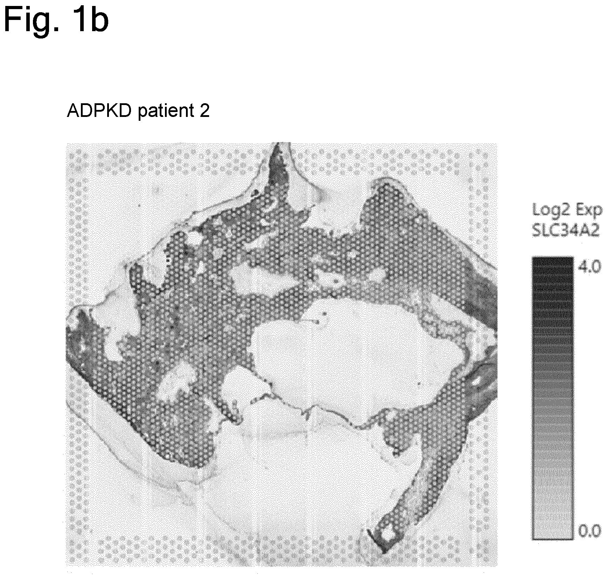

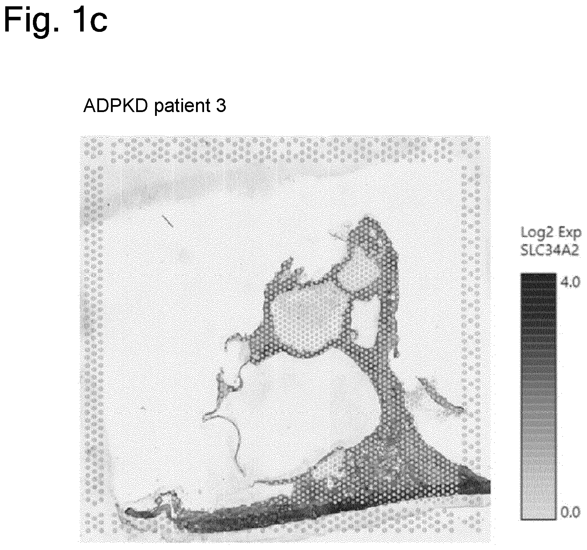

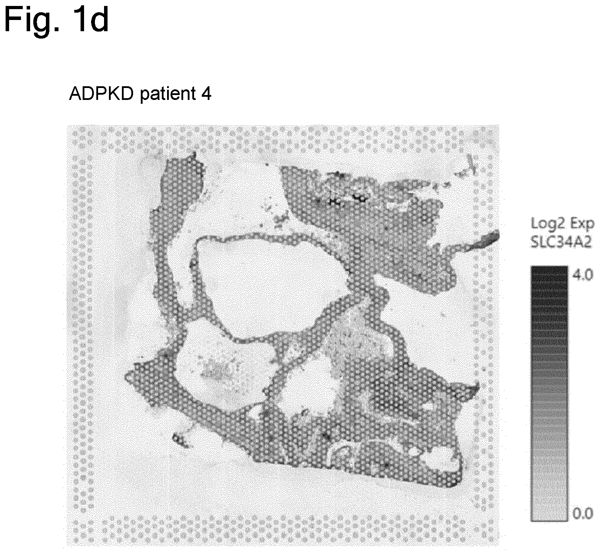

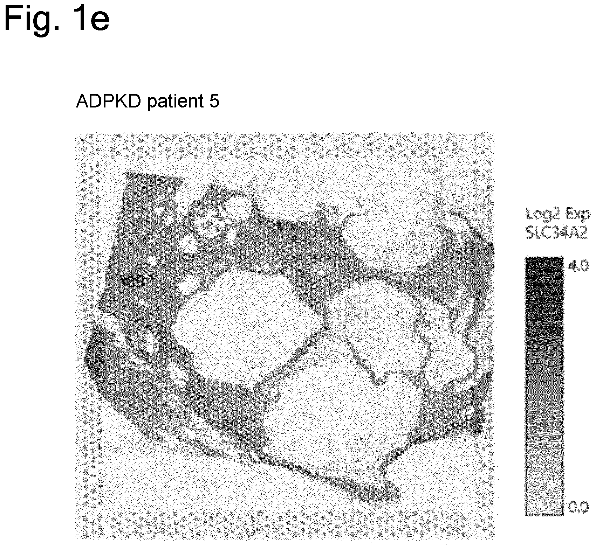

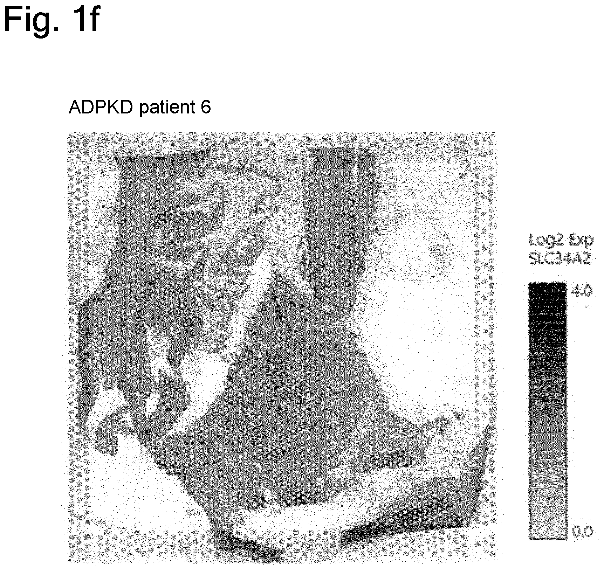

NaPi-IIb mRNA levels in kidney tissues of human ADPKD patients were evaluated using Visium Spatial Gene Expression (10x Genomics). Tissues containing cysts were excised from kidney tissues removed from ADPKD patients and frozen blocks were made by OCT embedding. The frozen blocks were sliced and put on slides for Visium Gene Expression, and HE staining was performed. After obtaining the HE staining image, cDNA was generated and sequence analysis of short read sequence library was performed for cDNA using NovaSeq 6000 (Illumina, Inc). The result of sequence analysis was reflected in the HE staining image using Loupe Browser (10x Genomics). The number of each gene in each spot calculated by the sequence analysis was corrected by the number of housekeeping genes and the number of nucleic acids in each spot to obtain a value of the expression level of each gene. The HE staining images and NaPi-IIb expression levels are shown in Figs. 1a to 1f. In Fig. 1, the grayscale bars show NaPi-IIb expression levels, where 4.0 indicates log₂2^{4.0}, and 0.0 indicates log₂2^{0.0}.

As the results of the above tests, highly expression of NaPi-IIb was confirmed in kidney tissues of human ADPKD patients.

Test Example 3: Evaluation of NaPi-IIb expression levels in kidney tissue of PKD model mouse

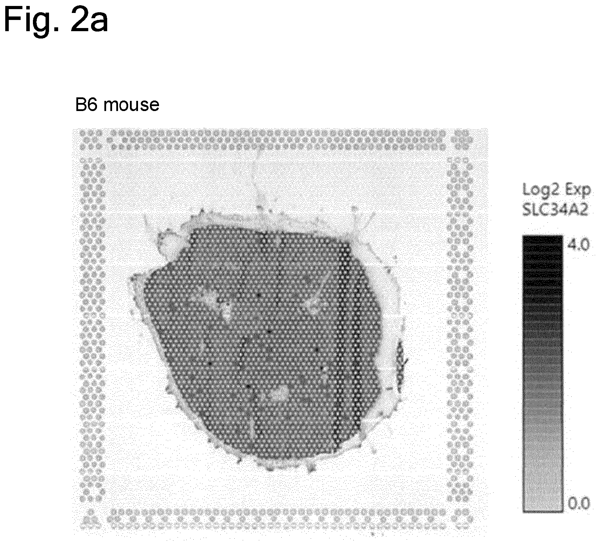

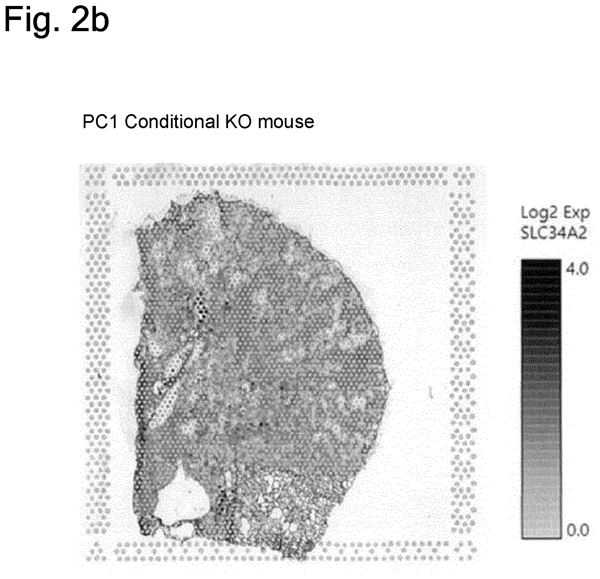

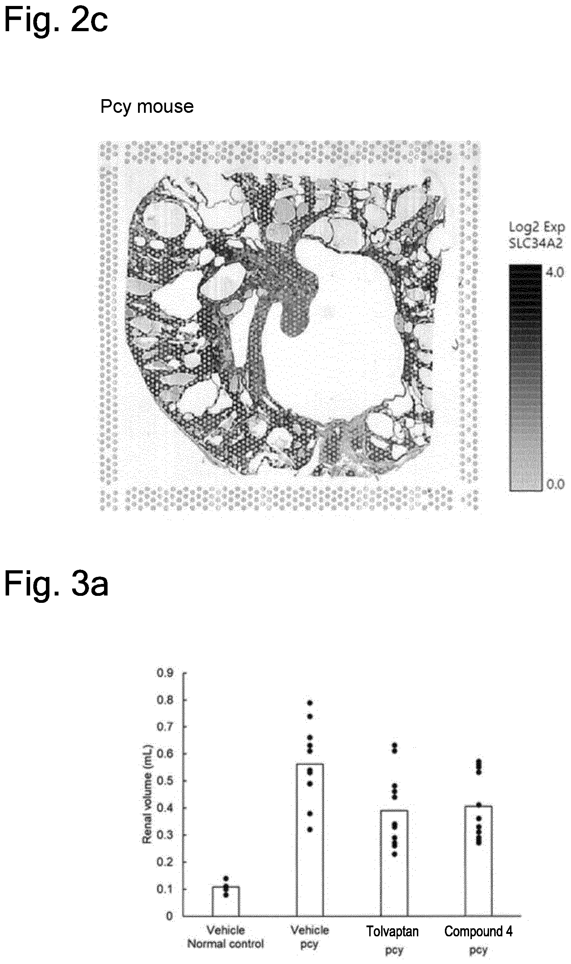

NaPi-IIb mRNA levels in kidney tissues of PC1 conditional knockout mice (obtained by passage breeding of those produced at Lieden University), DBA/2FG-pcy mouse (hereinafter referred to as pcy mouse) (KYUDO CO., LTD.) and C57BL/6J mice (CLEA Japan, Inc.) were confirmed using Visium Spatial Gene Expression (10x Genomics). Tamoxifen was orally administered to PC1 conditional knockout mice at a dose of 3 mg/kg for 3 days from day 21 after birth, and the kidneys were removed 16 weeks after administration. For DBA/2FG-pcy and C57BL/6J mice, kidneys were removed from 9-week-old individuals. Subsequent procedures were the same as in Test Example 2. Figs. 2a to 2c are diagrams showing HE staining images and NaPi-IIb expression levels of kidney tissues of PC1 conditional knockout mouse, pcy mouse, and C57BL/6J mice. In Figs. 2a to 2c, the grayscale bars show NaPi-IIb expression levels, where 4.0 indicates log₂2^{4.0}, and 0.0 indicates log₂2^{0.0}.

As the results of the above tests, highly expression of NaPi-IIb was confirmed in kidney tissues of mouse PKD models.

Test Example 4: Evaluation of suppression effects on increase in renal volume and decrease in renal function in pcy mice

The pcy mice, a naturally occurring PKD model, were used to evaluate the effects of tolvaptan and compound 4 in Test Example 1 on (a) renal volume, (b) blood urea nitrogen (UN) concentration, (c) body weight, and (d) urine volume in pcy mice.

The pcy mice (5 weeks of age at the start of this test) were grouped into 3 groups of 13 mice each. Compound 4 or tolvaptan was administered to each of the test compound administration groups. The administration period was 5 weeks. Compound 4 was administered subcutaneously once daily and tolvaptan was administered subcutaneously at a weekly pace. Compound 4 was administered at 33-40 mg/kg per dose and tolvaptan was administered at 25-30 mg/kg per dose. Compound 4 and tolvaptan were administered after dissolved in 50% DMSO/50% glycerol. A group of mice of the parental line was provided as a normal control group (Normal Control) (N = 7). The 50% DMSO/50% glycerol was administered to the normal control group and the Vehicle group of pcy mice at 1 mL/kg.

The left renal volume (renal volume (mL)) of mice was calculated by the following formula using echography 5 weeks after the start of administration of the test compound, as shown in Fig. 3a. Renal volume (mL) = π x long diameter (mm) x short diameter (mm) x short diameter (mm) x 1000/6

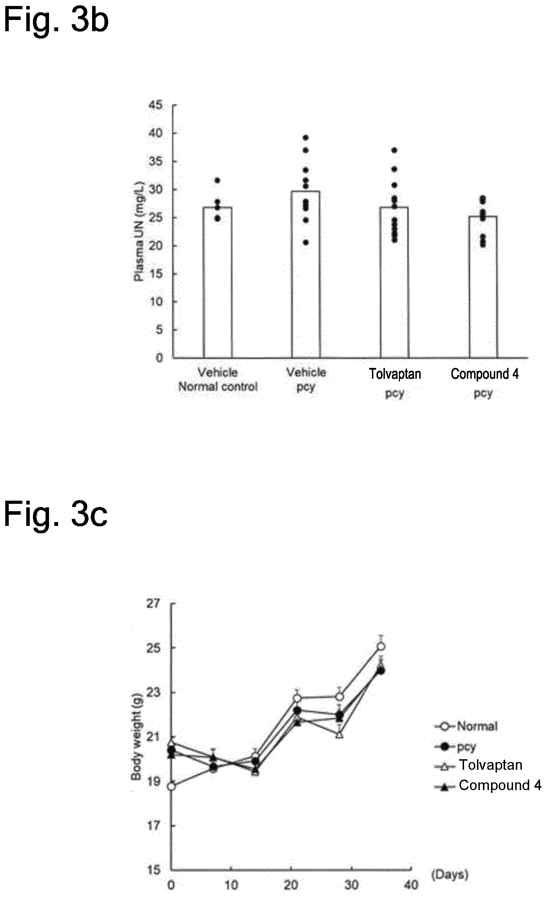

After that, blood was collected from the abdominal vena cava, plasma was collected, and euthanasia treatment was performed. Blood urea nitrogen (UN) concentration (Plasma UN (mg/L)) was measured using L-type Waco UN (Fujifilm Wako Pure Chemical Corporation), as shown in Fig. 3b.

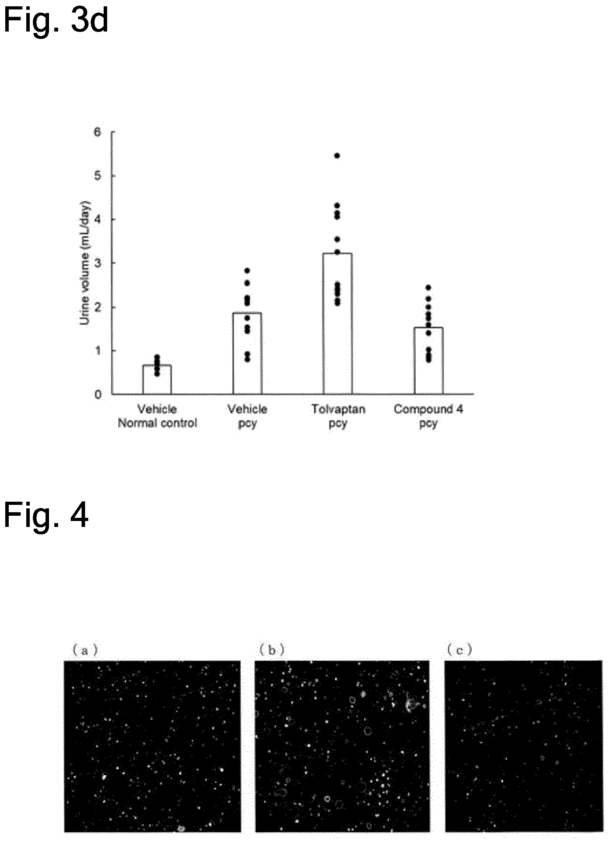

In addition, the body weight (Body weight (g)) of mice was measured every 7 days after the start of administration of the test compound, as shown in Fig. 3c. Furthermore, the volume of urine (Urine volume (mL/day)) was calculated from the specific gravity and measured weight of urine 5 weeks after the start of administration of the test compound, as shown in Fig. 3d.

The results of the above tests confirmed that compound 4 has the same degree of suppression effects on increase in renal volume and decrease in renal function as tolvaptan. It was also shown that compound 4 has less weight loss and no increase in urine volume compared to tolvaptan.

Test Example 5: Evaluation of inhibition effect on three-dimensional cyst formation using primary cells from kidney tissue of human ADPKD patient

Primary cells were isolated from kidney tissues of human ADPKD patients. Under two-dimensions, the isolated cells were cultured for 2 days using DMEM/F-12 (Thermo Fisher Scientific, Inc.) supplemented with 10% FBS. Subsequently, three-dimensional culture was performed for 7 days using 3D Ready Atelocollagen (KOKEN CO., LTD.). During three-dimensional culture, 50 µM forskolin (Sigma-Aldrich Co. LLC) and ITS liquid media supplements (Sigma-Aldrich Co. LLC) were added as stimulating agents to some of the wells to form cysts, and a test compound was added at the final concentration of 30 µM. The test compound was also added to other wells at the final concentration of 30 µM. After 7 days of three-dimensional culture, it was fixed with 4% paraformaldehyde (Thermo Fisher Scientific, Inc.), and nucleic acid staining and cell membrane staining were performed with Hoechst 33342 (Thermo Fisher Scientific, Inc.) and CellLight Plasma Membrane-RFP, BacMam 2.0 (Thermo Fisher Scientific, Inc.). Three-dimensional imaging and volume analysis were performed using Operetta CLS and image analysis software Harmony (PerkinElmer Japan G.K.). The inhibition rate was determined by the following formula.Inhibition rate (%) = (1 - (volume of stimulating agent and test compound-added well - volume of stimulating agent-added and test compound-non added well)/(volume of stimulating agent-added and test compound-non added well - volume of stimulating agent and test compound-non added well)) x 100

Fig. 4 shows three-dimensional photographed images of (a) stimulating agent and compound 4-non added well, (b) stimulating agent-added and compound 4-non added well, and (c) stimulating agent and compound 4-added well. Table 2 shows the average inhibition rate of each test compound (N = 3).

**[Table 2]**

| [Table 2: Results of Test Example 5] | |
|---|---|
| Compound No. | Inhibition rate (%) |
| 1 | 98.2 |
| 2 | 100.0 |
| 3 | 100.0 |
| 4 | 94.4 |

The results of the above tests confirmed the inhibitory effect of each test compound on high cyst formation.

Test Example 6: Evaluation of suppression effects on increase in renal volume and decrease in renal function in PC 1 conditional knockout mice

The PC1 conditional knockout mice, a PKD model, were used to evaluate the effects of tolvaptan and compound 4 in Test Example 1 on (a) renal volume, (b) body weight, and (c) urine volume in PC1 conditional knockout mouse.

Tamoxifen was orally administered to PC1 conditional knockout mice at a dose of 3 mg/kg for 3 days from day 21 after birth. The PC1 conditional knockout mice (5 weeks of age at the start of this test) were grouped into 3 groups of 15 mice each. Compound 4 or tolvaptan was administered as a single agent to the test compound administration group. The administration period was 16 weeks, and they were administered subcutaneously once daily. Compound 4 was administered at 33-40 mg/kg per dose and tolvaptan was administered at 25-30 mg/kg per dose. Compound 4 and tolvaptan were administered after dissolved in 50% DMSO/50% glycerol. A group of mice to which tamoxifen was not administered was provided as a normal control group (Normal Control) (N = 7). The 50% DMSO/50% glycerol was administered to the normal control group and the Vehicle group of PC1 conditional knockout mice at 1 mL/kg.

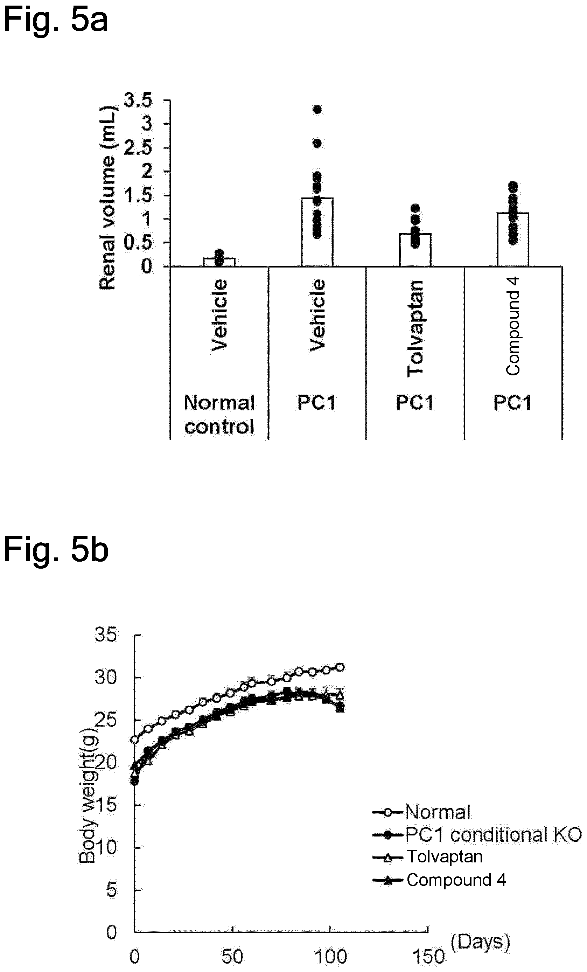

The left renal volume (renal volume (mL)) of mice was calculated by the following formula using echography 16 weeks after the start of administration of the test compound. The results are shown in Fig. 5a. Renal volume (mL) = π x long diameter (mm) x short diameter (mm) x short diameter (mm) x 1000/6

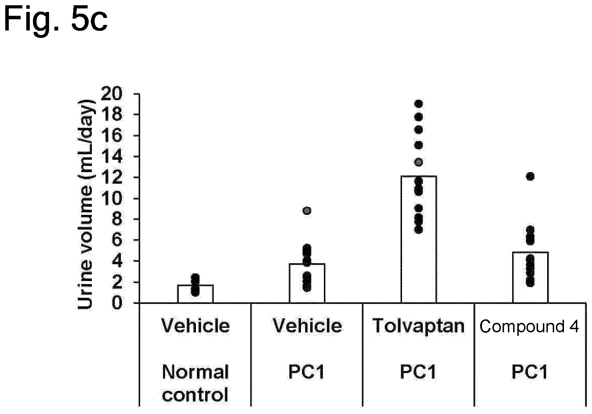

After that, euthanasia treatment was performed. In addition, the body weight (Body weight (g)) of mice was measured every 7 days after the start of administration of the test compound. The results are shown in Fig. 5b. Furthermore, the volume of urine (Urine volume (mL/day)) was calculated from the specific gravity and measured weight of urine 16 weeks after the start of administration of the test compound. The results are shown in Fig. 5c.

The results of the above tests confirmed the suppression effect of compound 4 on increase in renal volume and decrease in renal function. It was also shown that compound 4 has less weight loss and no increase in urine volume compared to tolvaptan.

## Claims

1. A pharmaceutical composition comprising a compound that inhibits a phosphate transporter as an active ingredient, for use in treating or preventing a cystic disease.

2. A pharmaceutical composition comprising a compound that inhibits a phosphate transporter as an active ingredient, for suppressing formation or expansion of a cyst.

3. The pharmaceutical composition according to claim 1 or 2, wherein the phosphate transporter is at least one selected from the group consisting of NaPi-IIa, NaPi-IIb, and NaPi-IIc.

4. The pharmaceutical composition according to any one of claims 1 to 3, wherein the phosphate transporter is NaPi-IIb.

5. The pharmaceutical composition according to any one of claims 1, 3 and 4, wherein the cystic disease is a disease involving NaPi-IIb.

6. The pharmaceutical composition according to any one of claims 2 to 4, wherein the cyst is a cyst involving NaPi-IIb.

7. The pharmaceutical composition according to any one of claims 1 to 6, wherein the compound is at least one selected from the group consisting of a low-molecular compound, a polypeptide, and a polynucleotide.

8. The pharmaceutical composition according to any one of claims 1 to 7, wherein the compound is a compound selected from the group consisting of compounds represented by formulas 1 to 11: 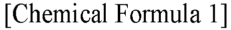 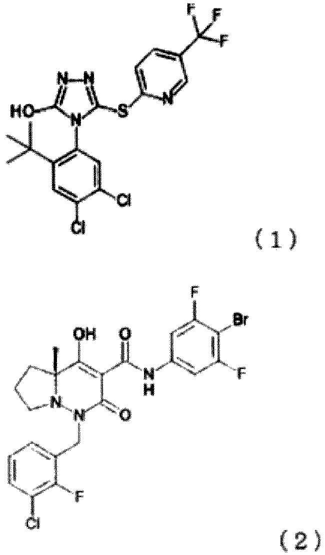 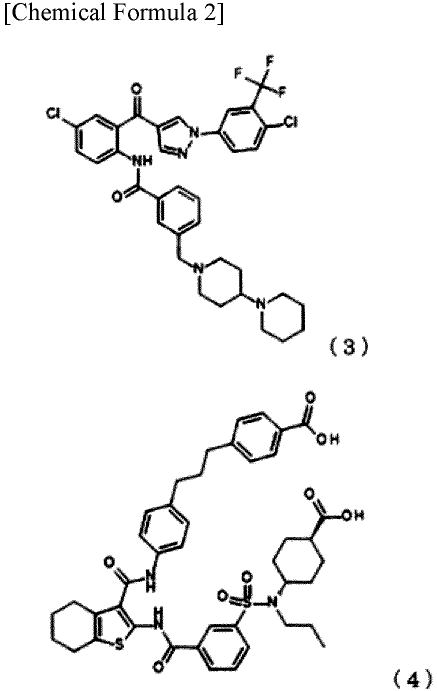 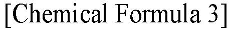 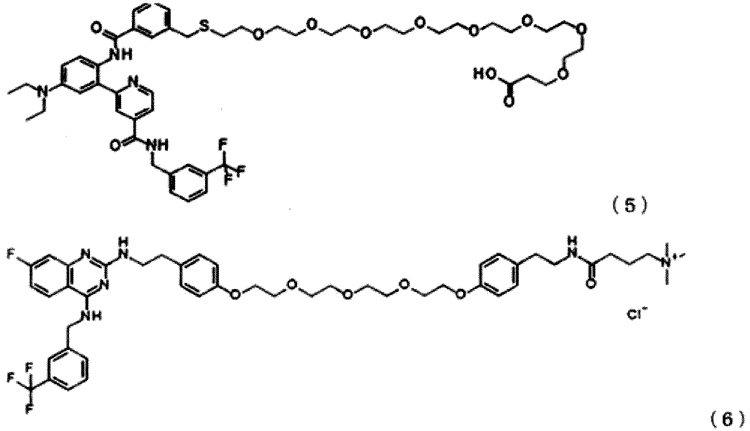 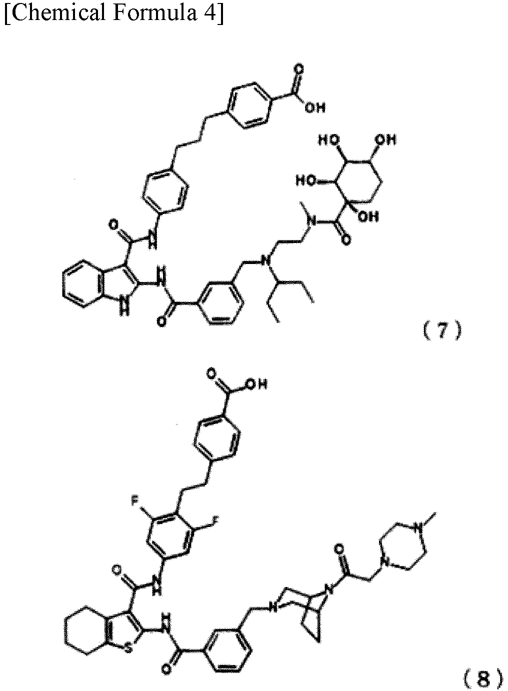 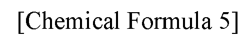 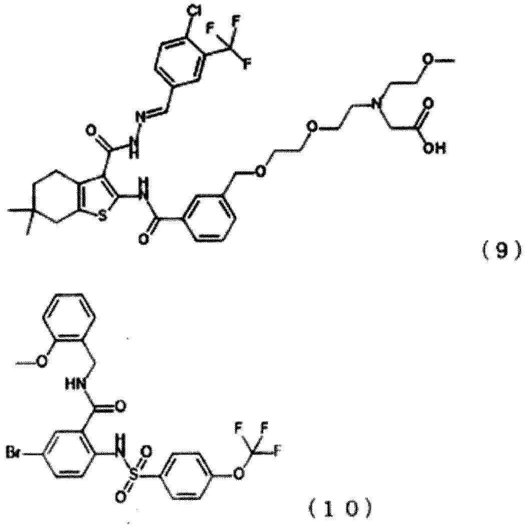 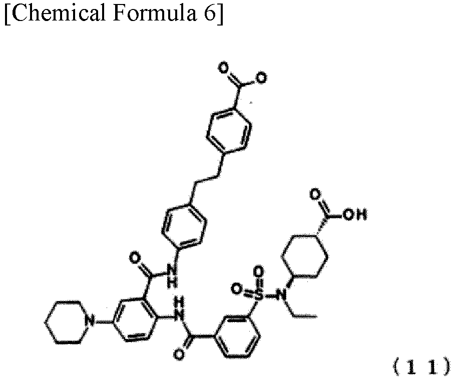 or a salt thereof, or a solvate thereof.

9. The pharmaceutical composition according to any one of claims 1 to 8, wherein the cystic disease is selected from the group consisting of cystic kidney disease, polycystic liver disease, cystic lung disease, and pancreatic cyst.

10. The pharmaceutical composition according to any one of claims 2 to 4 and 6 to 9, wherein the cyst is a cyst that is formed or expanded in a kidney, a liver, a lung, or a pancreas.

11. The pharmaceutical composition according to any one of claims 1 to 10, for use in treating or preventing cystic kidney disease.

12. The pharmaceutical composition according to any one of claims 1 to 11, for use in treating or preventing polycystic kidney disease.

13. A method for predicting a benefit of a treatment with an agent in a patient having a cystic disease, the method comprising:
confirming whether NaPi-IIb is expressed in a cell collected from an organ of the patient in which a cyst has been formed; and
determining the patient as a patient who would benefit from the treatment when the expression is confirmed,
wherein the agent is an inhibitor of NaPi-IIb.

14. A method of screening for a compound for use in treating or preventing a disease selected from cystic kidney disease, polycystic liver disease, cystic lung disease, and pancreatic cyst, the method comprising measuring an inhibitory activity against a phosphate transporter.

15. A method of screening for a compound that suppresses formation or expansion of a cyst in an organ of a mammal, the method comprising measuring an inhibitory activity against a phosphate transporter.
